# EUROPEAN PATENT SPECIFICATION

(11) **EP 3 405 265 B1**
(45) Date of publication and mention of the grant of the patent: **08.09.2021**
(21) Application number: 17742051.0
(22) Date of filing: 20.01.2017
(51) Int. Cl.: G16H 20/30, A63B 21/04, A63B 71/00, A63B 21/02, A63B 21/05, A63B 23/02, A63B 23/04, A63B 23/12, A63B 21/00, A63B 71/06, A63B 21/055, A63B 23/035

(54) **SYSTEMS AND METHODS FOR SIMULTANEOUSLY CONTRACTING BODY CORE**
SYSTEME UND VERFAHREN FÜR GLEICHZEITIG KONTRAKTIERENDEN KÖRPERKERN
SYSTÈMES ET PROCÉDÉS DE CONTRACTION SIMULTANÉE DE NOYAU CORPOREL

(30) Priority: 20.01.2016 US 201662281136 P; 04.10.2016 US 201662404125 P
(43) Date of publication of application: 28.11.2018
(73) Proprietor: Core 46 IP LLC, Irvine, CA 92618 (US)
(72) Inventor: HOEVEN, M., Arnold, Irvine CA 92618 (US)
(74) Representative: Witte, Weller & Partner Patentanwälte mbB
(86) International application number: PCT/US2017/014417
(87) International publication number: WO 2017/127735

(56) References cited:
- WO-A1-94/09855
- WO-A1-99/32195
- US-A- 5 085 429
- US-A- 5 492 524
- US-A- 5 697 873
- US-A- 6 056 676
- US-A1- 2002 111 252
- US-A1- 2008 090 706
- US-A1- 2011 275 498
- US-A1- 2014 243 153

## Description

### Technical Field

The present disclosure relates generally to exercise equipment.

### Background

Currently, 90% of American's do not regularly strength train. Part of the reason includes the lack of devices that are convenient to use and accomplish maximum results in the least amount of time. The permanent, gradual muscle loss starting at age 25 has resulted in a new area of medicine referred to as sarcopenia. In addition, the cost due to lower back pain has doubled in the last 20 years from $50 billion to over $100 billion in the United States alone. This level is expected to exceed $220 billion by 2025.

One of the causes of lower back pain is weak and permanent loss of core muscles. Many home and professional exercise machines focus on only a single muscle group at once, which lack the ability to isolate and strengthen multiple interconnected muscles. Many of these machines require significant time at the gym and have the potential to increase the risk of muscle injury. Many of these machines also lack convenience and portability.

Document US 2002/111252 A1, according to its abstract, relates to an exercise device including a hollow body having openings into which a user may repeatedly push vertical and horizontal legs. The vertical leg has a saddle at its top for engagement with the chest of a user and contains a bellows for resisting the user's efforts to push the vertical leg into the hollow body with his chest. The horizontal legs have cradles at their outer ends for engaging the thighs of a user and contain bellows for resisting the user's efforts push the horizontal legs into the hollow body with his thighs. Valves associated with the bellows permit the force required to push the vertical and horizontal legs into the hollow body to be varied.

Document US 6 056 676 A, according to its abstract, relates to an exercise device for facilitating the strengthening of abdominal and back muscles. The exercise device includes a resistance member coupled between a lap engageable base and an arm positioning member. The arm positioning member helps to maintain the back of the user in a substantially upright position as the arm positioning member applies and releases reciprocatively and alternatingly in a substantially vertical path of travel of the upper body of the user against the force on the resistance member during repetitions of use of the device. The resistance member resists substantially vertical downward movement relative to the base member to enhance the development of muscle strength by enabling the upper body of the user to contract and expand reciprocatively vertically without leaning forwardly in a safe and effective manner. The exercise device may have at least one swivel connecting the arm positioning member to the base that allows the exercise device to be used while the user leans left or leans right, thus exercising auxiliary back muscles. Further, the swivel and a torso adjustment assembly allow the user to maintain a comfortable, safe, and effective exercise position.

Document US 5 697 873 A, according to its abstract, relates to an exercise device comprising an elongate strut on which a pair of limb engaging members are located. At least one of the limb engaging members is slidable along the length of the strut towards and away from the other member. A resistive force means such as one or more elastomeric bands links the movable limb engaging member to a reaction point on the strut or the other limb engaging member. The arrangement is such that movement of the movable limb engaging member along the strut in at least one direction requires work to be done against the action of the resistive force means. Preferably both limb engaging members are movable along the length of the strut towards and away from each other. The strut may be formed in more than one section with the different ends of the sections engageable with each other to provide for different modes of operation of the device.

### Summary

There is a need for exercise devices that can simultaneously exercise multiple core muscle groups, often within only 3-5 minutes. Therefore, there are provided an exercise device according to claim 1 and a method according to claim 11. As an exemplary advantage, such a device could combine multiple mechanical power units and simultaneously coordinate multiple biomechanical movements or multiple anatomical muscle groups. Such a device would simultaneously contract or strengthen all core upper and lower muscle in a fluid harmony. Simultaneous exercise can require the user to produce more total effort and energy as the user coordinates the movement of multiple muscle groups compared to working out each muscle group alone. Exercising multiple muscles simultaneously builds muscle strength more effectively, improves flexibility, reduces the time needed to achieve results, decreases the risk of potential injury, and alleviates back pain.

The exercise apparatus includes a first thigh member and a second thigh member moving in a first reciprocal motion when the user engages the first and second thigh members, the first reciprocal motion varying, or adapted to vary, a first distance between the first and second thigh members, and a chest contact member moving, or adapted to move, in a second reciprocal motion when a user engages the chest contact member, the second reciprocal motion varying, or adapted to vary, a second distance between the chest contact member and the first and second thigh members. In some embodiments, simultaneously varying the first distance and second distance can compound the exercise benefits of varying the first distance and the second distance sequentially or separately. In some embodiments, simultaneously varying the first distance and second distance can require the user to produce more total effort and energy as the user coordinates the movement of multiple muscle groups compared to working out each muscle group alone. In some embodiments, simultaneously varying the first distance and second distance can build more strength and burn more calories than sequential or separate motion.

In some embodiments, the exercise apparatus further includes a first resistance member, wherein the first resistance member provides, or is adapted to provide, a restoring force on the first thigh member relative to the second thigh member when the first distance decreases. In some embodiments, a maximum distance between the first and second thigh members is adjustable.

In some embodiments, the first reciprocal motion includes both the first thigh member and the second thigh member moving, or adapted to move, relative to a centerline of the exercise apparatus. In some embodiments, the exercise apparatus further includes a second resistance member, wherein the second resistance member provides, or is adapted to provide, a restoring force on the chest contact member when the second distance decreases.

According to the invention the exercise apparatus further includes an abdominal contact member moving, or adapted to move, in a third reciprocal motion when the user engages the abdominal contact member, the third reciprocal motion varying, or adapted to vary, a third distance between the abdominal contact member and the first and second thigh members. In some embodiments, the apparatus further includes an abdominal crunch base guiding, or adapted to guide, the abdominal contact member when a user engages the abdominal contact member and moves it in the third reciprocal motion. In some embodiments, the exercise apparatus further includes a third resistance member, wherein the third resistance member provides, or is adapted to provide, a restoring force on the abdominal contact member when the third distance decreases.

In some embodiments, the first and second thigh members are configured to move in a fourth reciprocal motion when the user engages the first and second thigh members, the fourth reciprocal motion varying, or adapted to vary, a fourth distance between the first and second thigh members and the chest contact member. In some embodiments, the exercise apparatus further includes a fourth resistance member, wherein the fourth resistance member provides, or is adapted to provide, a restoring force on the one or more of the first and second thigh members relative to the chest contact member when the fourth distance decreases.

In some embodiments, the restoring force of one or more of the resistance members is adjustable. In some embodiments, one or more of the resistance members are interchangeable with other resistance members.

In some embodiments, the exercise apparatus further includes one or more handles. In some embodiments, one or more of the back, upper, lower and side core muscle groups contact member and the chest contact member further includes one or more handles.

The invention also relates to a method of exercising using an exercise apparatus, which includes engaging a first thigh member and a second thigh member, engaging a chest contact member, wherein a chest crunch includes the chest contact member, overcoming a restoring force provided by a first resistance member to vary a first distance between the first and second thigh members to move the first and second thigh members in a first reciprocal motion, and overcoming a restoring force provided by a second resistance member to vary a second distance between the chest contact member and one or more of the first and second thigh members to move the chest contact member in a second reciprocal motion, wherein overcoming the restoring force provided by the first resistance member is performed simultaneously with overcoming the restoring force provided by the second resistance member.

According to the invention, the method also includes engaging an abdominal contact member, and overcoming a restoring force provided by a third resistance member to vary a third distance between the abdominal contact member and one or more of the first and second thigh members to move the abdominal contact member in a third reciprocal motion, wherein overcoming the restoring force provided by the third resistance member is performed simultaneously with overcoming the restoring force provided by the first and second resistance members.

In some embodiments, overcoming the restoring force provided by the second resistance member or the third resistance member further includes engaging one or more handles to pull the exercise apparatus towards a user's body.

In some embodiments, the method includes overcoming a restoring force provided by a fourth resistance member to vary a fourth distance between one or more of the first and second thigh members and chest contact member to move one or more of the first and second thigh members in a fourth reciprocal motion, wherein overcoming the restoring force provided by the fourth resistance member is performed simultaneously with overcoming the restoring force provided by the first and second resistance members.

In some embodiments of the method of exercising, the restoring force of one or more of the resistance members are adjustable. In some embodiments of the method of exercising, the method includes adjusting one or more of the resistance members to vary the restoring force. In some embodiments of the method of exercising, one or more of the resistance members are interchangeable with other resistance members. In some embodiments of the method of exercising, the method includes interchanging one or more of the resistance members with other resistance members.

In some embodiments, a resistance member includes a first anchor; a second anchor configured to move in a reciprocal motion relative to the first anchor; a spring resisting relative movement of the first and second anchor in a first direction of the reciprocal motion and aiding relative movement of the first and second anchor in a second direction of the reciprocal motion opposite to the first direction; a spacer that limits relative motion of the first and second anchor in the first direction to a minimum distance between the first and second anchor, wherein the spring is in tension when the first and second anchor are at the minimum distance.

In some embodiments, the spacer is configured to maintain a distance between the first anchor and the second anchor of between 0.3175 cm and 50.8 cm [1/8 of an inch and 20 inches], between 0.635 cm and 45.72 cm [1/4 of an inch and 18 inches], between 1.27 cm and 40.64 cm [1/2 of an inch and 16 inches], between 2.54 cm and 35.56 cm [1 inch and 14 inches], between 5.08 cm and 30.48 cm [2 inches and 12 inches], between 7.62 cm and 20.32 cm [3 and 8 inches], between 8.89 cm and 15.24 cm [3.5 and 6 inches], or between 10.16 cm and 12.7 cm [4 and 5 inches].

In some embodiments, the spacer is configured to maintain a distance between the first anchor and the second anchor of 0.3175 cm, 0.635 cm, 1.27 cm 2.54 cm, 5.08 cm, 7.62 cm, or 10.16 cm [1/8, 1/4, or 1/2 of an inch, or 1, 2, 3, or 4 inches].

In some embodiments, the spring comprises latex or rubber.

In some embodiments, the spring comprises a continuous band encircling a first band connector and a second band connector.

In some embodiments, the first band connector is connected to the first anchor and the second band connector is connected to the second anchor.

In some embodiments, the resistance member includes a housing comprising the first anchor, second anchor, and the spring, wherein a length of housing is between 5.08 cm and 50.8 cm [2-20 inches], between 6.35 cm and 30.48 cm [2.5 and 12 inches], between 7.62 cm and 20.32 cm [3 and 8 inches], between 8.89 cm and 15.24 cm [3.5 and 6 inches], or between 10.16 cm and 12.7 cm [4 and 5 inches], a width of between 2.54 cm and 15.24 cm [1 and 6 inches], between 3.81 cm and 10.16 cm [1.5 and 4 inches], or between 5.08 cm and 7.62 cm [2 and 3 inches], and a height of between 1.27 cm and 7.62 cm [0.5 and 3 inches], or between 2.54 cm and 5.08 cm [1 and 2 inches].

In some embodiments, the housing comprises a length of 10.16 cm [4 inches], a width of 5.08 cm [2 inches], and a height of 2.54 cm [1 inch].

In some embodiments, the housing comprises a first housing comprising the first anchor and a second housing comprising the second anchor, wherein at least one of the first and second anchors comprise an outer surface of the first and second housing.

In some embodiments, the resistance member includes a third anchor fixed relative to one of the first and second anchor and configured to move in a reciprocal motion relative to the other of the first and second anchor.

In some embodiments, the third anchor comprises a protrusion from one of the first and second housings.

In some embodiments, the resistance member includes a fourth anchor fixed relative to the third anchor.

In some embodiments, the fourth anchor comprises a protrusion from one of the first and second housings.

In some embodiments, the first anchor is configured to engage a chest contact member and the second anchor is configured to engage a chest crunch member.

In some embodiments, the first anchor is configured to engage an abdominal contact member and the second anchor is configured to engage an abdominal crunch member.

In some embodiments, the first anchor is configured to engage a first thigh member and the second anchor is configured to engage a second thigh member.

In some embodiments, an exercise apparatus includes a body contact member; and a base, wherein one of the body contact member and the base comprises a tube and the other comprises an insert configured to move in a reciprocal motion within the tube, the tube comprises a hollow internal space comprising an inner wall, the insert comprises an outer wall, the reciprocal motion comprises the inner wall facing, and moving relative, to the outer wall, and the tube and the insert comprise complimentary guides to prevent contact between the inner wall and outer wall during the reciprocal motion.

In some embodiments, the complementary guides comprise a bearing and a female guide rail.

In some embodiments, the complementary guides reduce an angular range of motion of tube relative to the insert.

In some embodiments, an exercise apparatus includes a body contact member; and a base, wherein one of the body contact member and the base comprises a tube and the other comprises an insert, wherein the insert moves in a reciprocal motion within the tube, wherein the insert comprises a first anchor engagement and the tube comprises a second anchor engagement, wherein the first anchor engagement moves relative to the second anchor engagement, and wherein the tube comprises an aperture sized to receive a resistance member that engages with the first anchor engagement and the second anchor engagement.

In some embodiments, a resistance member includes a first spring connector; a second spring connector, wherein a first distance between the first spring connector and second spring connector is fixed; a spring connected to the first spring connector and the second spring connector; and a paddle configured to engage the spring, wherein the paddle is configured to move in a reciprocal motion relative to the first and second anchors, and wherein the spring resists relative movement between the paddle and the first and second anchors in a first direction of the reciprocal motion and aids relative movement between the paddle and the first and second anchors in a direction of the reciprocal motion opposite the first direction.

In some embodiments, the spring comprises a continuous band encircling the first spring connector and the second spring connector.

In some embodiments, the spring comprises latex or rubber.

In some embodiments, the reciprocal motion is between 5.08 cm and 45.72 cm [2 and 18 inches], between 10.16 cm and 40.64 cm [4 and 16 inches], between 12.7 cm and 30.48 cm [5 and 12 inches], or between 15.24 cm and 20.32 cm [6 and 8 inches].

In some embodiments, the reciprocal motion is 2.54 cm, 5.08 cm, 7.62 cm, 10.16 cm, 12.7 cm, 15.24 cm, 17.78 cm, 20.32 cm, or 22.86 cm [2, 3, 4, 5, 6, 7, 8, or 9 inches].

In some embodiments, one of an abdominal contact member or an abdominal crunch base comprises or is connected to the paddle and the other of the abdominal crunch member or abdominal crunch bases comprises or is connected to the first and second spring connector.

In some embodiments, one of a chest contact member or a chest crunch base comprises or is connected to the paddle and the other of the chest crunch member or chest crunch bases comprises or is connected to the first and second spring connector.

In some embodiments, a first thigh member comprises or is connected to the paddle and a second thigh member comprises or is connected to the first and second spring connector.

### Brief Description of the Drawings

Fig. 1A shows an isometric view of an exemplary apparatus, in accordance with an embodiment.
Fig. 1B shows a front view of the exemplary apparatus of Fig. 1A, in accordance with an embodiment.
Fig. 1C shows a side view of the exemplary apparatus of Fig. 1A, in accordance with an embodiment.
Fig. 1D shows a top view of the exemplary apparatus of Fig. 1A, in accordance with an embodiment.
Fig. 2A shows an isometric view of an exemplary apparatus, in accordance with an embodiment.
Fig. 2B shows a side view of the exemplary apparatus of Fig. 2A, in accordance with an embodiment.
Fig. 3A shows an isometric view of an exemplary apparatus, in accordance with an example which is not part of the invention.
Fig. 3B shows a side view of the exemplary apparatus of Fig. 3A, in accordance with an example which is not part of the invention.
Fig. 4 shows an isometric view of an exemplary apparatus, in accordance with an embodiment.
Fig. 5A shows an isometric view of an exemplary apparatus, in accordance with an embodiment.
Fig. 5B shows a side view of an exemplary apparatus, in accordance with an embodiment.
Fig. 6A and Fig. 6B show a method of using an exemplary apparatus, in accordance with an embodiment.
Fig. 7A and Fig. 7B show a method of using an exemplary apparatus, in accordance with an embodiment.
Fig. 8A and Fig. 8B show a method of using an exemplary apparatus, in accordance with an embodiment.
Figure 9A and Figure 9B represent isometric cutaway views of an exemplary resistance member in accordance with an embodiment.
Figure 9C illustrates a resistance member in accordance with another embodiment.
Figure 9D illustrates a resistance member in accordance with another embodiment.
Figure 10A and Figure 10B represent top-down views of an exemplary resistance member compartment in accordance with an embodiment.
Figure 10C and Figure 10D represent top-down views of an exemplary resistance member placed inside an exemplary resistance member compartment.
Figure 10E illustrates a resistance member compartment without a resistance member inserted, in accordance with an embodiment.
Figure 10F illustrates a resistance member compartment without a resistance member inserted, in accordance with an embodiment.
Figure 11 represents an isometric view of a disassembled exemplary resistance member 1100 in accordance with an embodiment.
Figure 12 represents an isometric view of a disassembled exemplary resistance member 1200 in accordance with an embodiment.
Figures 13A and 13B illustrate paddles configured to be used with the springs of Figures 11 and 12.
Figure 14 illustrates an exercise apparatus comprising a body contact member and a base.
Figures 15A - 15F illustrate exemplary contact members and backings for abdominal contact members and chest contact members.

### Detailed Description

Machines and devices can make exercise and rehabilitation of an illness or injury more effective and more efficient. Such devices can precisely isolate specific muscle groups during a workout, improve a user's form, and provide additional resistance.

In the following description of preferred embodiments, reference is made to the accompanying drawings which form a part hereof, and in which it is shown by way of illustration specific embodiments in which the invention can be practiced. It is to be understood that other embodiments can be used and structural changes can be made without departing from the scope of the embodiments of this invention. Any of the embodiments described above or in the following can be combined with one or more embodiments and/or with one or more elements from one or more embodiments.

Some embodiments of the devices and methods disclosed herein involve combining four different exercises: 1) thigh adduction, 2) lateral resistance pulls; 3) direct resistance to the front abdominals comparable to doing crunches slowly with a weighted medicine ball; and 4) a decline bench crunch. Some embodiments of the devices and methods disclosed herein involve combining the following four different exercises: 1) thigh abduction, 2) lateral resistance pulls; 3) direct resistance to the front abdominals comparable to doing crunches slowly with a weighted medicine ball; and 4) a decline bench crunch. The exercises can be completed simultaneously in a coordinated and fluid manner. In some embodiments, the resistance the device supplies for each exercise is individually adjustable to suit the user.

As an exemplary advantage, the devices and methods described herein can simultaneously exercise multiple core muscle groups, thus increasing workout efficiency, building muscle strength, and improving flexibility.

Currently, there are no exercise devices that simultaneously contract all core muscles and the thigh adductors. A strong core can be the foundation to a better, healthier body. The core provides the foundation for virtually all muscle movement. The upper core includes all the muscles of the midsection and hips that support the spine, back, and neck, while the lower core includes the hip connection and glutes. Developing a strong core, including strong abdominal muscles, can help prevent back injury and pain by improving posture and spinal alignment, alleviate the amount of work other muscle groups must do to bear the weight of the entire body, and improve balance and stability.

The same is true of adductor muscles. The adductor muscles work in concert with the abdomen, back, and shoulder muscles to stabilize the torso. Weak adductor muscles can increase the chances of knee, ankle, and foot injuries. Simultaneously exercising the core and adductor muscles means that these muscles are working together as they do in normal, natural motion.

Working out core and hip adductor muscle groups simultaneously has the added benefit of compounding the effects of each individual exercise. Simultaneous exercise can require the user to produce more total effort and energy as the user coordinates the movement of multiple muscle groups compared to working out each muscle group alone. A user exercising both core and adductor muscles can overcome a greater total opposing force and burn more calories than exercising the groups individually. This can increase the total intensity of a workout and build stronger muscles with greater efficiency.

For example, a user exercising multiple muscle groups simultaneously can use more weight or resistance and burn more calories than working out a single muscle group in isolation. Working out core muscles separately can significantly reduce the intensity of the contractions and resistance compared to multiple simultaneous contractions. Typically, greater levels of core muscle contraction lead to increased fast twitch muscle fiber recruitment. This is why more resistance, less repetitions and slower anaerobic movements can be more conducive to strengthening the core muscles. Many abdominal/back machines incorporate fast movements resulting in very little strengthening of the abdominal/back fast twitch fiber muscles. As a result, an individual could be better off doing eighteen crunches slowly with a 13.61 kg (30-pound) medicine ball, as opposed to 300 crunches fast.

Building muscle strength typically requires a person to frequently and consistently exercise the same muscles over and over. Given how busy people are, the compounding effects of simultaneous exercise become increasingly important. Machines that isolate individual muscle groups do not allow people to maximize their workout of each muscle group. Machines that isolate individual muscle groups also do not allow people return to each muscle group often enough to see gains in muscle size. Exercising multiple muscle groups at once allows a user to exercise more muscle groups more often, and therefore see a resulting gain in total muscle size and strength.

Another exemplary advantage includes focusing on multiple muscle groups simultaneously, thereby reducing the amount of time a person needs to complete a full-body exercise routine. This allows a person to exercise more muscle groups in the same limited period of time.

As another exemplary advantage, the devices and methods described herein assist a user in completing the exercises with proper posture and body position. Proper posture and alignment can be important in preventing injury and maximizing a workout. As another exemplary advantage, a user can perform the exercises described herein in a variety of orientations, including while seated or supine. As another exemplary advantage, the systems and methods described herein allow a user to mimic the mechanics of the traditional exercises described above while adding external resistance.

Another exemplary advantage includes the ability to perform exercises at varying speeds and with varying levels of resistance. Certain exercises may be more effective when performed in slow, controlled motions or when performed in rapid bursts. As previously discussed, greater levels of core muscle contraction lead to increased fast twitch muscle fiber recruitment. Thus, more resistance, fewer repetitions and slower anaerobic movements can be more conducive to strengthening the core muscles. In some embodiments, the devices and methods disclosed herein incorporate slow movements resulting in greater strengthening of the abdominal or back fast twitch fiber muscles. As a result, an individual could be better off doing a few repetitions slowly using the devices and methods described herein than doing a large number of unassisted crunches quickly.

Because the devices and methods described herein provide an efficient workout, a user can frequently and consistently return to exercising the same muscle groups often enough in subsequent workouts to increase muscle size.

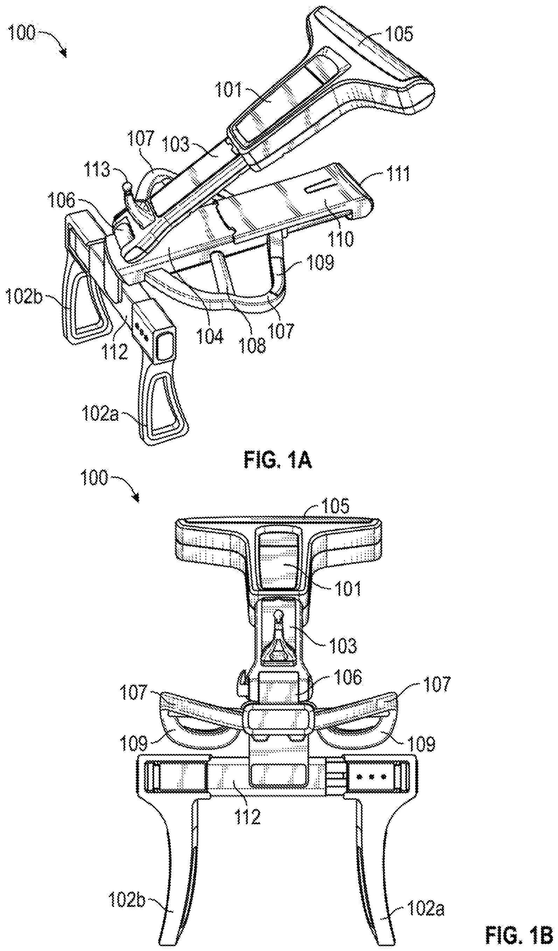

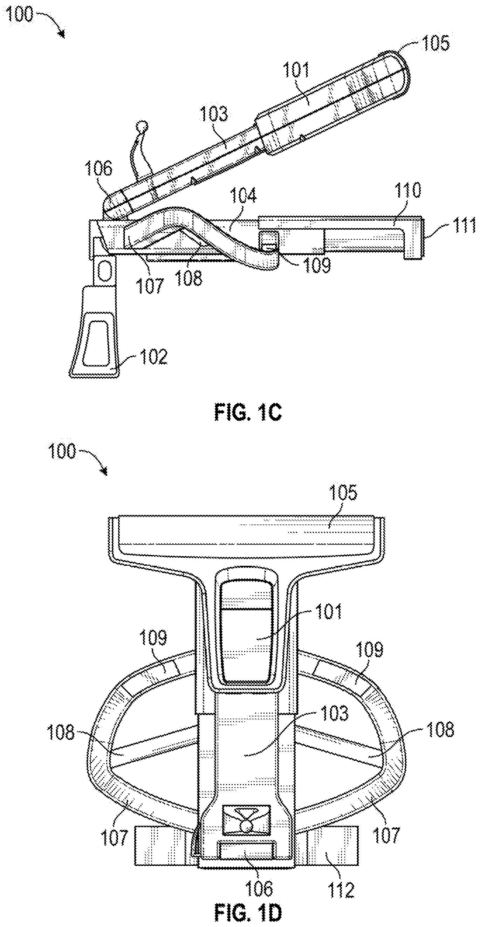

Figure 1A represents an isometric view of an exemplary exercise apparatus 100 in accordance with an embodiment. Figures 1B, 1C, and 1D represent front, side, and top views of apparatus 100, respectively. Apparatus 100 allows a user to perform a thigh adduction exercise while simultaneously contracting many or all twenty-four segmented abdominal muscles (within the rectus, oblique and serratus groups), lats and back flexox muscles. These muscles collectively constitute the core muscle groups. The exercise apparatus 100 includes a first thigh member 102a and a second thigh member 102b and a chest contact member 101. As will be described, the first thigh member 102a and second thigh member 102b can help a user perform a thigh adduction exercise. The chest contact member 101 can allow the user perform an exercise equivalent to a decline crunch. In some embodiments, the chest contact member 101 is coupled to a chest crunch base 103.

Apparatus 100 includes an abdominal crunch contact member 110, which can provide direct resistance to the front abdominals comparable to doing crunches slowly with a weighted medicine ball resting on the abdomen.

The adductor muscles exercised can include the adductor brevis, adductor longus and adductor magnus, for example. Exercising these muscles can include a user starting with his or her thighs spaced apart, contracting his or her thighs towards a midline of the user's body, and then releasing the adductor muscles to return the thighs to the starting position. Thus, in some embodiments, apparatus 100 assists in exercising thigh adductor muscles by providing additional resistance during thigh adduction.

The first thigh member 102a can be connected to the second thigh member 102b such that the first and second thigh members are configured to move in a reciprocal motion. The reciprocal motion includes varying a distance between the first thigh member 102a and second thigh member 102b.

In some embodiments, the exercise apparatus contains a resistance member that is configured to provide a restoring force on the first thigh member 102a relative to the second thigh member 102b when the distance between the first and second thigh members decreases. In some embodiments, a maximum distance between 102a and 102b is 40.64 cm [16 inches], as measured from a point on 102a that is closest to a point on 102b. In some embodiments, the maximum distance is 38.1 cm [15 inches], 35.56 cm [14 inches], or 33.02 cm [13 inches]. In some embodiments, the maximum distance between 102a and 102b is configured such that the maximum distance between a user's knees while still in contact with thigh members 102a and 102b is between 35.56 cm and 60.96 cm [14 and 24 inches], including 58.42 cm [23 inches], 55.88 cm [22 inches], 53.34 cm [21 inches], 50.8 cm [20 inches], 48.26 cm [19 inches], 45.72 cm [18 inches], 43.18 cm [17 inches], 40.64 cm [16 inches], and 38.1 cm [15 inches]. In some embodiments, the maximum and or minimum distance between the first thigh member 102a and second thigh member 102b is adjustable. Thus, in some embodiments the device can accommodate multiple users of various sizes or multiple thigh adduction exercises. For example, the user can choose between short, quick movements with a smaller maximum distance between the first thigh member 102a and second thigh member 102b, or slower, longer movements across a wider range of motion.

In some embodiments, the device is configured to exercise a user's abductor muscles. As will be apparent to a person of ordinary skill in the art, the first thigh member 102a and second thigh member 102b can be configured to engage the outside of a user's thighs while moving in a reciprocal motion. The reciprocal motion includes varying a distance between the first thigh member 102a and second thigh member 102b. In some embodiments, the exercise apparatus contains a resistance member that is configured to provide a restoring force on the first thigh member 102a relative to the second thigh member 102b when the distance between the first and second thigh members increases.

In some embodiments, the first thigh member 102a and second thigh member 102b can rotate about an axis such that the thigh members can be configured or positioned to engage the inside or outside of a user's thighs depending upon the position of the thigh members, or at any angle in between that accommodates a user's thighs. In some embodiments, the thigh members include a locking mechanism to secure the thigh members in place.

In some embodiments, both the first thigh member 102a and second thigh member 102b move in a reciprocal motion relative to a fixed point, such as a centerline of apparatus 100. In some embodiments, a centerline of apparatus 100 can be understood to be a line between the first thigh member and second thigh member, whether equidistant from the first thigh member and second thigh member, or not. In some embodiments, a centerline of apparatus 100 can be understood to be an element of apparatus 100 between the first thigh member and second thigh member, such as abdominal crunch base 104 (described more fully below).

In some embodiments, the first thigh member 102a and second thigh member 102b are attached to a thigh base 112. Therefore, in some embodiments, both the first thigh member 102a and second thigh member 102b can move relative to a point fixed at the center of the thigh base 112. In some embodiments, one of the first thigh member 102a or second thigh member 102b is fixed at a set distance from the fixed point, and the other of the first thigh member 102a or second thigh member 102b moves relative to the fixed point. In some embodiments, one of the first thigh member 102a or second thigh member 102b is in a fixed position on the thigh base 112, while the other of the first thigh member 102a or second thigh member 102b slides along the thigh base 112 in a reciprocal motion relative to the fixed thigh member or the centerline of apparatus 100.

In some embodiments, the first and second thigh members 102a and 102b, respectively, are contoured to receive and engage a user's thighs. The thigh members can have a concave shape or a "C" or "U" shape. The shape can be designed with a radius of curvature to more closely contour to the shape of the human thigh. The curvature can allow a user's thighs to nest within the thigh member in a manner that increases the surface area of the thigh member contacting the user's thighs. Increasing the contact area can increase a user's comfort. The size of the thigh members can also vary to increase the amount of surface area in contact with a user's thighs. In some embodiments, the thigh members have a wide contact area to distribute the force of the exercise across a larger part of the user's thighs. The thigh members 102a and 102b can include padding or cushioning.

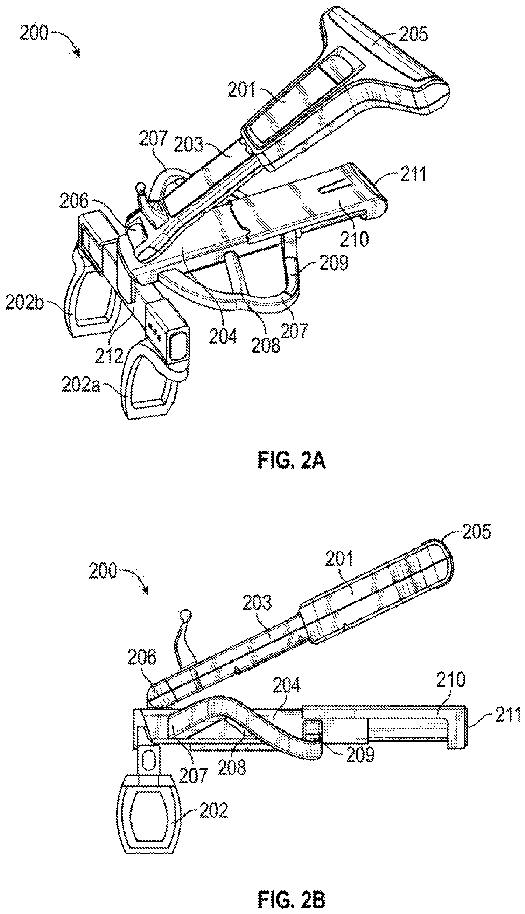

Figure 2A and Figure 2B represent an isometric and side view, respectively, of an exemplary exercise apparatus 100 in accordance with an embodiment. In some embodiments, the thigh members (202a and 202b) contain one or more contact areas surrounding a hollow area. The distance between the contact areas surrounding a hollow area can vary. The one or more contact areas can include bars or tubes of various sizes, as well. The contact areas may be more rounded to mimic the shape of a thigh. Thigh members 202a and 202b can be used with any of the embodiments here, including replacing thigh members 102a, 102b, 302a, 302b, 402a, and 402b.

In some embodiments, a width of a thigh member 102a or 102b can be between 5.08 cm and 30.48 cm [2 and 12 inches], including between 10.16 cm and 20.32 cm [4-8 inches], including 15.24 cm [6 inches]. In some embodiments, the width of a thigh member is not uniform, but can vary at different points of the thigh member. In some embodiments, a height of a thigh member 102a or 102b is between 10.16 cm and 30.48 cm [4 and 12 inches], including 17.78 cm, 20.32 cm, or 22.86 cm [7, 8, or 9 inches]. In some embodiments of a curved or concave thigh member, the top portion of thigh members 202a and 202b that are adapted to contact the top of a user's thighs can have a length of between 5.08 cm and 25.4 cm [2 and 10 inches], including 12.7 cm, 15.24 cm, 17.78 cm, 20.32 cm, or 22.86 cm [5, 6, 7, 8 or 9 inches].

As an exemplary advantage, the user can exercise his or her thigh adductors in nearly any position, including while seated, lying on his or her back, or while performing any of the other exercises described herein, including while performing a crunch. In some embodiments, the first thigh member 102a is parallel to the second thigh member 102b. In some embodiments, the first thigh member 102a is not parallel to the second thigh member 102b. For example, in some embodiments, the first and second thigh members are angled such that a distance between a side of the thigh members closer to the user is smaller than a distance between a side of the thigh members further from the user. In some embodiments, the angles of the first thigh member 102a and second thigh member 102b approximate the angle of a user's thighs when a user first engages the first thigh member 102a and second thigh member 102b. In some embodiments, the angles of the first thigh member 102a and second thigh member 102b approximate the angle of a user's thighs when a user varies the distance between the first thigh member 102a and second thigh member 102b.

Returning to Figures 1A-1D, in some embodiments, apparatus 100 provides direct resistance to a user's chest comparable to doing a decline bench crunch, which can target the rectus abdominus and iliopsoas muscles. The chest contact member 101 can be configured to move in a reciprocal motion relative to the chest crunch base 103, the thigh members (102a and/or 102b), or an abdominal crunch base 104 when a user engages the chest contact member. The reciprocal motion can include varying a distance between the chest contact member and the chest crunch base 103, the thigh members (102a and/or 102b), or the abdominal crunch base 104. The reciprocal motion can also include being adapted to vary a distance between the chest contact member and the chest crunch base 103, the thigh members (102a and/or 102b), or the abdominal crunch base 104.

In some embodiments, a resistance member provides a restoring force on the chest contact member 101 relative to the chest crunch base 103, the thigh members (102a and/or 102b), or the abdominal crunch base 104 when the distance decreases. Thus, increasing the resistance can increase the force the abdominal muscles must exert to overcome it. As an exemplary advantage, increasing resistance can improve the effectiveness or efficiency of the workout. As another exemplary advantage, providing resistance to the chest can allow a user to perform the equivalent of a decline crunch while in a seated position or while lying in a flat and level position.

In some embodiments, the resistance member is included in the connection between the chest contact member 101 and the chest crunch base 103, the thigh members (102a and/or 102b), and/or the abdominal crunch base 104.

The chest contact member 101 can be connected to the chest crunch base 103 such that the reciprocal motion includes the chest contact member 101 sliding over or inside of the chest crunch base 103 and guiding the chest crunch member 101 during the reciprocal motion. The chest contact member 101 can include a contact surface for engaging a user's chest 105. In some embodiments, the surface 105 is large enough to increase stability and user comfort during use of apparatus 100. The surface can include a chest pad, such as a foam cushion. The chest pad can be interchangeable with pads of other sizes, shapes and materials to suit a particular user or exercise. In some embodiments, the chest pad is smooth. In some embodiments, the chest pad is textured. In some embodiments, the texture is configured to increase friction between the surface 105 and the user's body to reduce slipping during use.

In some embodiments, the apparatus can be modular. In some embodiments, a chest contact member and a chest crunch base can be part of a chest module, an abdominal contact member and an abdominal contact base can be part of an abdominal module, and a first thigh member and a second thigh member can be part of a thigh module. In some embodiments, one or more of the modules can be detachable from the apparatus. In some embodiments, one of more of the modules can be interchangeable on the apparatus. In some embodiments, one or more of the modules can be permanently affixed to the apparatus. In some embodiments, the chest contact member 101 is detachable from the remaining portions of the device. This may include detaching a chest crunch base 103. Thus, the chest contact member 101 and chest crunch base may comprise a modular component that can be connected to the thigh members or the abdominal contact/base member. In the same way, the thigh members may be detachable and the abdominal contact member may be detachable.

In some embodiments, a length of a chest module can be between 30.48 cm and 76.2 cm [12 and 30 inches], including 50.8 cm [20 inches]. In some embodiments, the length of the chest contact member can be between 15.24 cm and 76.2 cm [6 and 30 inches], including 20.32 cm, 30,.48 cm, 40.64 cm, and 50.8 cm [8, 12, 16, and 20 inches]. In some embodiments, the length of a chest crunch base can be between 15.24 cm and 76.2 cm [6 and 30 inches], including 20.32 cm, 30.48 cm, 40.64 cm, 50.8 cm, and 60.96 cm [8, 12, 16, 20, and 24 inches].

In some embodiments, a length of an abdominal module can be between 30.48 cm and 76.2 cm [12 and 30 inches], including 50.8 cm or 55.88 cm [20 inches or 22 inches]. In some embodiments, the length of the abdominal contact member can be between 15.24 cm and 76.2 cm [6 and 30 inches], including 20.32 cm, 25.4 cm, 30.48 cm, 40.64 cm, 50.8 cm, 55.88 cm, and 60.96 cm [8, 10, 12, 16, 20, 22, and 24 inches]. In some embodiments, the length of an abdominal crunch base can be between 15.24 cm and 76.2 cm [6 and 30 inches], including 20.32 cm, 25.4 cm, 30.48 cm, 40.64 cm, 50.8 cm, and 60.96 cm [8, 10, 12, 16, 20, 24 inches].

In some embodiments, a length of a proximal end of a chest contact member to a distal end of a chest crunch base is 40.64 cm, 45.72 cm, 50.8 cm, 55.88 cm, or 60.96 cm [16, 18, 20, 22, or 24 inches] when the chest contact member and the chest crunch base are coupled and in an extended position. In some embodiments, a length of a proximal end of a chest contact member to a distal end of a chest crunch base is 20.32 cm, 25.4 cm, 30.48 cm, 35.56 cm, or 40.64 cm [8, 10, 12, 14, or 16 inches] when the chest contact member and the chest crunch base are coupled and in a compressed position. In some embodiments, the ratio of a (1) length of a proximal end of a chest contact member to a distal end of a chest crunch base when the chest contact member and the chest crunch base are coupled and in an extended position to (2) a length of a proximal end of the chest contact member to a distal end of the chest crunch base when the chest contact member and the chest crunch base are coupled and in a compressed position is 2:1, 5:3, or 3:2.

In some embodiments, a length of a proximal end of an abdominal contact member to a distal end of an abdominal crunch base is 40.64 cm, 45.72 cm, 50.8 cm, 55.88 cm, or 60.96 cm [16, 18, 20, 22, or 24 inches] when the abdominal contact member and the abdominal crunch base are coupled and in an extended position. In some embodiments, a length of a proximal end of an abdominal contact member to a distal end of an abdominal crunch base is 20.32 cm, 25.4 cm, 30.48 cm, 35.56 cm, or 40.64 cm [8, 10, 12, 14, or 16 inches] when the abdominal contact member and the abdominal crunch base are coupled and in a compressed position. In some embodiments, the ratio of (1) a length of a proximal end of an abdominal contact member to a distal end of an abdominal crunch base when the abdominal contact member and the abdominal crunch base are coupled and in an extended position to (2) a length of a proximal end of the abdominal contact member to a distal end of an abdominal crunch base when the abdominal contact member and the abdominal crunch base are coupled and in a compressed position is 2:1, 5:3, or 3:2.

In some embodiments, the ratio of (1) a length of a proximal end of an abdominal contact member to a distal end of an abdominal crunch base when the abdominal contact member and the abdominal crunch base are coupled and in an extended position to (2) a length of a proximal end of a chest contact member to a distal end of a chest crunch base when the abdominal contact member and the abdominal crunch base are coupled and in an extended position is 1:1. If the ratio deviates from a 1:1 ratio in the extended, contracted, or partially contracted positions, use can become awkward or uncomfortable. In particular, if the ratio is too low in any of these positions, the user can experience undue upward slippage of the chest contact member relative to their chest.

In some embodiments, the chest crunch base 103 is connected to the abdominal crunch base 104 by a hinge 106. In some embodiments, a main body of the apparatus comprises the chest crunch base 103, the abdominal crunch base 104, the hinge 106, and the thigh base 112. In some embodiments, the chest contact member / chest contact base can be detachably connected. In some embodiments, the height or angle of the chest contact member 101 relative to the thigh members can be adjusted, for example, by the hinge 106 connected to chest crunch base 103.

The user can adjust the height or angle of the chest contact member to accommodate different exercises or different users. In some embodiments, the height or angle of the chest contact member can be reversibly locked in a desired position using a lock, such as a quick release lever 117. Some embodiments include a quick release mechanism that is conveniently manipulated without great strain and with one hand, or just the force from one or a few fingers, so the other hand is free to hold the apparatus while the user completes the adjustment. In some embodiments, the user can adjust the angle of the chest contact member relative to the abdominal contact member at any angle between 0° (for example, parallel to the abdominal contact member) and 90° (for example, greater perpendicular to the abdominal contact member). In some embodiments, the user can set the angle of the chest contact member relative to the abdominal contact member at 0°, 10°, 20°, 30°, 40°, 45°, 50°, 60°, 70°, 80°, or 90°. In some embodiments, the user can set the angle of the chest contact member relative to the abdominal contact member at 30°, 40°, 45°, or 50°.

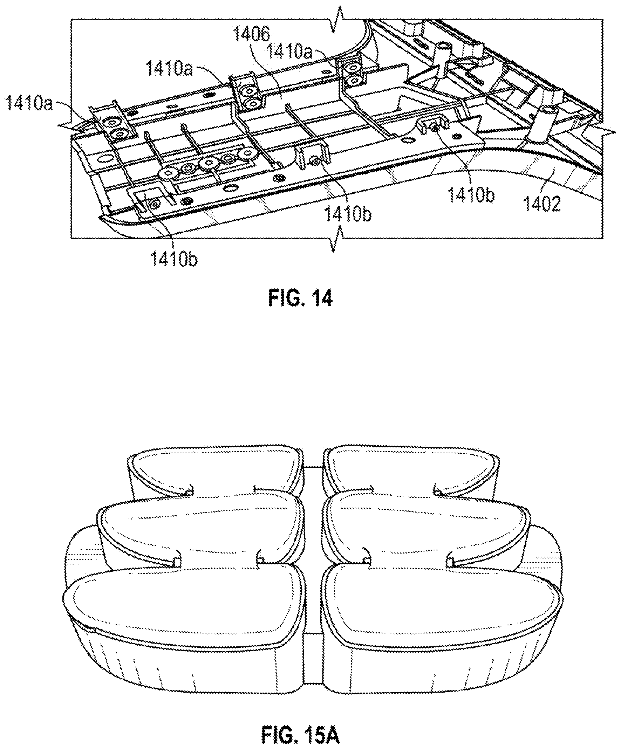

Apparatus 100 can also assist a user in performing a lateral resistance pull. This exercise can target the back muscles, including latissimus dorsi (lats), teres major, and trapezius muscles. The abdominal crunch base 104 can be generally rectangular. The abdominal crunch base 104 can include one or more handles 107. The one or more handles 107 can be positioned on one or more sides of the abdominal crunch base 104. Each handle 107 can include one or more handgrips 108 or arm supports 109. Apparatus 100 also includes an abdominal contact member 110. The abdominal contact member 110 is configured to move in a reciprocal motion relative to the abdominal crunch base 104 when a user engages the abdominal contact member 110. Thus, the user can perform a lateral resistance pull by engaging the handgrips 108 with the user's hands and pulling the abdominal crunch base 104 towards the user's abdomen in a reciprocal motion. The reciprocal motion can include varying a distance, or being adapted to vary a distance, between the abdominal crunch base 104 and the abdominal contact member 110, the chest crunch base 103, and/or the thigh members (102a and 102b). In some embodiments, apparatus 100 can include a resistance member that is configured to provide a restoring force on the abdominal contact member 110 when the user moves the abdominal crunch base 104 in a reciprocal motion. In some embodiments, the resistance member is included in the connection between the abdominal contact member 110 and the abdominal crunch base 104, the thigh members (102a and/or 102b), and/or the chest crunch base 103. Exemplary abdominal contact members and backings for abdominal contact members are provided in Figures 15A - 15F.

In some embodiments, apparatus 100 provides direct resistance to the front abdominals comparable to doing crunches slowly with a weighted medicine ball resting on the abdomen. This exercise can target the middle and upper abdominal muscles, the lower back, and the core. The abdominal contact member can include a contact surface 111 for engaging a user's abdomen. The contact surface 111 can be of any size. In some embodiments, the surface 111 is large enough to increase stability and user comfort during use of the apparatus. The surface 111 can include a pad, such as a foam cushion. The pad can be interchangeable with pads of other sizes, shapes and materials to suit a particular user or exercise. As the distance between the abdominal contact member 110 and the abdominal crunch base 104 decreases, the amount of pressure the contact surface 111 places on the abdomen can increase.

Various configurations of the apparatus are possible, provided that they fall within the scope of the claims. For example, different configurations can comprise different combinations of components. The combinations can include some or all of the components described above. Adding or withdrawing components can provide for different exercises, isolation of different muscle groups, or different methods of use. Thus, in some embodiments, the modular components of the device can be used independently, or combined in various configurations to provide for customized workouts.

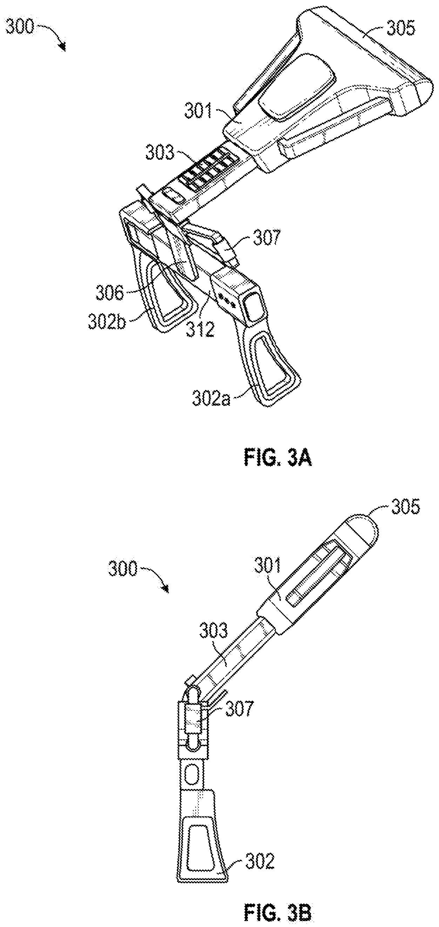

Figure 3A represents an isometric view of an exemplary exercise apparatus 300 in accordance with an example which is not part of the invention. Figure 3B represents a side view of an exemplary exercise apparatus 300 in accordance with an example which is not part of the invention. Apparatus 300, like apparatus 100 described above, includes a chest contact member 301 and a first thigh member 302a and a second thigh member 302b. In apparatus 300, the first thigh member 302a can be connected to the second thigh member 302b such that the first thigh member 302a and second thigh member 302b are configured to move in a reciprocal motion. The reciprocal motion can include varying a distance, or being adapted to vary a distance, between the first thigh member 302a and second thigh member 302b. In some embodiments, the exercise apparatus contains a resistance member that is configured to provide a restoring force on the first thigh member 302a relative to the second thigh member 302b when the distance between the first and second thigh members decreases.

In some embodiments, the chest contact member 301 can be configured to move in a reciprocal motion relative to the chest crunch base 303 or the thigh members (302a and/or 302b) when a user engages the chest contact member. The reciprocal motion can include varying a distance, or being adapted to vary a distance, between the chest contact member 301 and the chest crunch base 303 or the thigh members (302a and/or 302b). In some embodiments, a resistance member provides a restoring force on the motion of the chest contact member 301.

The chest crunch base 303 is optionally connected to the thigh base 312 by a hinge 306. In some embodiments, a main body of apparatus 300 comprises the chest crunch base 303, the hinge 306, and the thigh base 312.

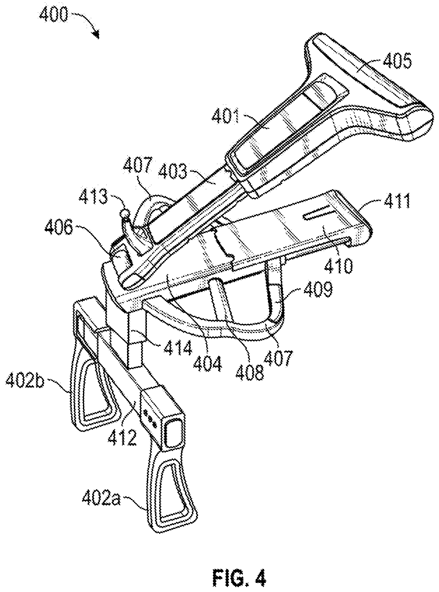

The device can be further configured to provide additional functionality, such as a leg-raise unit. Figure 4 represents an isometric view of an exemplary exercise apparatus 400 in accordance with an embodiment. Thigh base 412 can be configured to move in a reciprocal motion relative to the abdominal crunch base 404 and/or the chest contact member 401 in some embodiments. The user can vary the distance between the thigh base 412 and the abdominal crunch base 404 and/or the chest contact member 401 by engaging the thigh members 402a and 402b and raising his or her legs while simultaneously engaging at least one of the abdominal contact member 410 and the chest contact member 401. In some embodiments, a resistance member 414 is configured to provide a restoring force on the thigh base 412 relative to the abdominal crunch base 404 and/or the chest contact member 401 when the distance between the base and the abdominal crunch base decreases. In some embodiments, the resistance member 414 provides additional resistance and/or increases the distance that can be varied before, during, or after a crunch exercise.

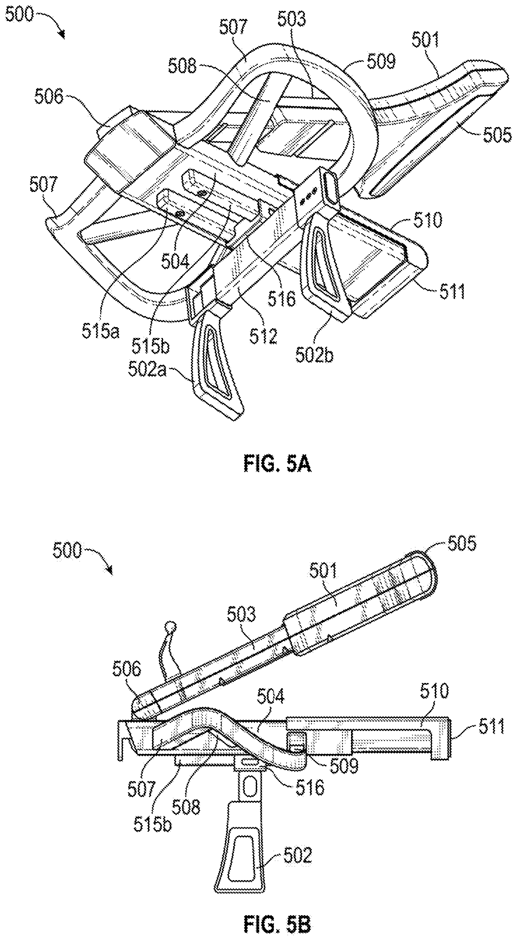

In some embodiments, the device is configured such that the leg unit is not fixed to a particular position of the apparatus. Figure 5A represents an isometric view of an exemplary exercise apparatus 500 in accordance with an embodiment. Figure 5B represents a side view of an exemplary exercise apparatus 500 in accordance with an embodiment. In some embodiments, it can be advantageous to allow the thigh members to remain in a fixed or relatively fixed position along a user's thigh while using the apparatus. Therefore, in some embodiments, the thigh base 512 is configured to attach and slide relative to the abdominal crunch base 504 or the abdominal contact member 510. In some embodiments, the abdominal crunch base 504 or the abdominal contact member 510 comprises one or more rails. The rails can be bars, tracks, or grooves that allow the first and second thigh members to move relative to the abdominal crunch base 504 or the abdominal contact member. In some embodiments, the thigh base further comprises a sliding member 516. In some embodiments, the sliding member is mounted on the one or more rails 515a and 515b.

The resistance members described herein can be interchangeable with other resistance members. In some embodiments, one or more resistance members can be or can comprise a tension band, a cord, hydraulics, weights, compression bands, or springs. In some embodiments, a resistance member can comprise a plurality of resistance members. In some embodiments, the resistance member can be adjusted to vary the amount of resistance provided. For example, an adjustable resistance member can provide between 0.45 kg and 68.04 kg [1 and 150 pounds] of resistance. In some embodiments, extra resistance members can be stored in a chamber contained within the device when not in use. The chamber can be in the chest contact member, chest crunch base, or the abdominal contact member, for example.

In some embodiments, the apparatus includes an electronic computer, such as an electronic personal trainer or electronic coaching device as described in U.S. Patent Nos. 7,717,825 and 7,955,220. The electronic coaching device could be implemented as a separate device. In some embodiments, the computer is removably affixed to the abdominal crunch base 104 via a ball and socket joint 113. In some embodiments, the computer can include a smart phone or tablet. In some embodiments, the smart phone or tablet can run an app. The computer can include an electronic (audio and visual) personal trainer that assists in the technique and training time of the exercise. The user can choose beginner or intermediate/advanced workout sessions or settings. The total time for a workout session can range from 1-20 minutes, 2-10 minutes, or 3-4 minutes, for example.

In some embodiments, the apparatus can comprise a biometric authentication device, including, for example, a fingerprint reader, retina or iris scanner, or voice identification device, to authenticate a user or users. In some embodiments, the electronic personal trainer uses the biometric device to detect and identify an individual user or users. In some embodiments, the apparatus comprises sensors and/or timers that measure the force, time, count, and/or distance of each reciprocal motion. In some embodiments, the electronic personal trainer can store and/or analyze data for one or more users of the apparatus, including data generated by the one or more sensors and/or timers.

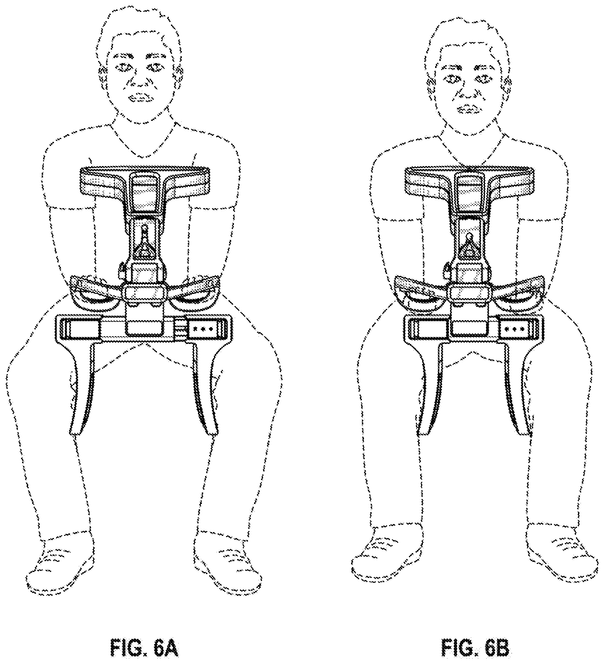

Figure 6, Figure 7, and Figure 8 illustrate exemplary methods of using the apparatus in accordance with various embodiments of the present disclosure. The exercise methods of Figures 6-8 can be used to perform exercises using the various apparatuses disclosed herein, but the methods are not limited to using the various apparatuses disclosed herein. In some embodiments, where a modular component is not included in an apparatus, the corresponding motion in Figures 6-8 is not performed.

In some embodiments, a method of exercising using an exercise apparatus includes engaging a first thigh member and a second thigh member, engaging a chest contact member, overcoming a restoring force provided by a first resistance member to vary a first distance between the first and second thigh members to move the first and second thigh members in a first reciprocal motion, and overcoming a restoring force provided by a second resistance member to vary a second distance between the chest contact member and at least one of the first and second thigh members to move the chest contact member in a second reciprocal motion, wherein overcoming the restoring force provided by the first resistance member is performed simultaneously with overcoming the restoring force provided by the second resistance member.

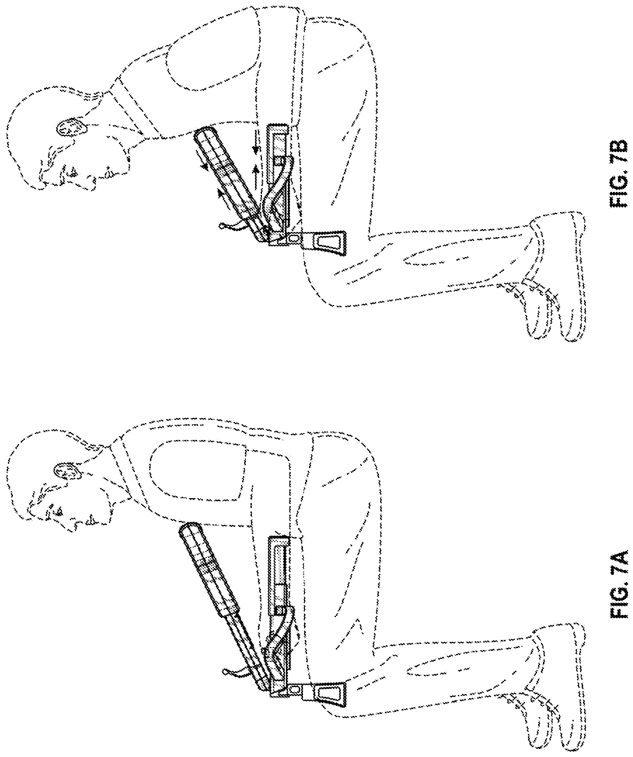

Figures 6A and 6B depict an exemplary method of varying a distance between the first and second thigh members in a reciprocal motion. Figures 7A and 7B depict an exemplary method of varying a distance between the chest contact member and at least one of the first and second thigh members to move the chest contact member in a reciprocal motion. Figures 7A and 7B also depict the user varying a distance between the abdominal contact member and at least one of the first and second thigh members to move the abdominal contact member in a reciprocal motion. This motion can simulate the use of an abdominal medicine ball when the device is pulled toward the user in the lateral pull motion, described above. Also as previously described, the first and second thigh members can be attached to the apparatus at a fixed position in some embodiments. Figures 7A and 7B show the thigh members sliding along the user's thighs as the user pulls the apparatus towards his abdomen in a lateral pull.

In some embodiments, the method of exercising includes engaging an abdominal crunch member comprising an abdominal contact member and overcoming a restoring force provided by a resistance member to vary a distance between the abdominal contact member and at least one of the first and second thigh members to move the abdominal contact member in a reciprocal motion. Figures 7A and 7B also depict an exemplary method of varying a distance between the abdominal contact member and at least one of the first and second thigh members to move the chest contact member in a reciprocal motion. In some embodiments, the method of exercising includes engaging an abdominal contact member and overcoming restoring force provided by a resistance member to vary a distance between the abdominal contact member and an abdominal crunch base to move the abdominal contact member in a reciprocal motion.

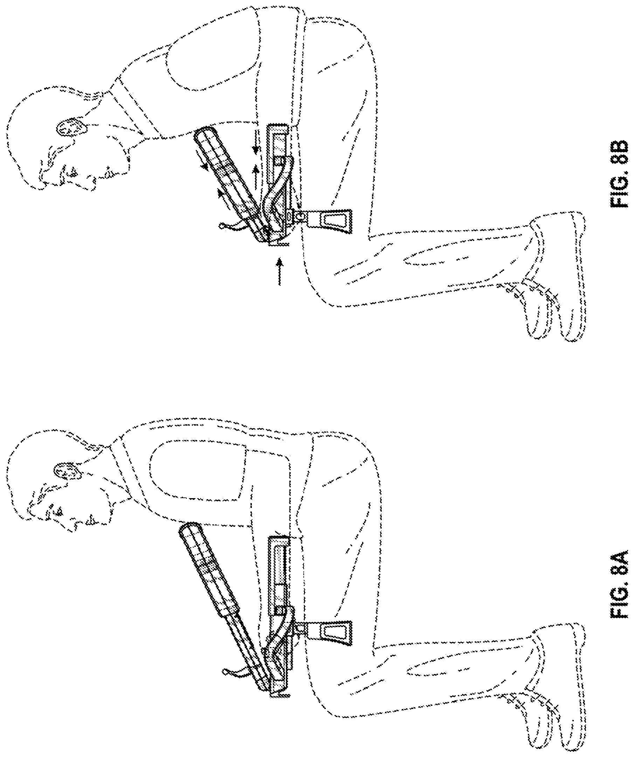

Figures 8A and 8B depict an exemplary method of varying a distance between the chest contact member and at least one of the first and second thigh members to move the chest contact member in a reciprocal motion. Figures 8A and 8B also depict the user varying a distance between the abdominal contact member and the abdominal crunch base to move the abdominal contact member in a reciprocal motion. This motion can simulate the use of an abdominal medicine ball when the device is pulled toward the user in the lateral pull motion, described above. Also as previously described, the first and second thigh members can be attached to the apparatus such that the first and second thigh members slide along the abdominal crunch base or abdominal contact member in some embodiments. Figures 8A and 8B show the thigh members sliding along the abdominal crunch base and remaining in a fixed position relative to the user's thighs as the user pulls the apparatus towards his abdomen in a lateral pull.

In some embodiments, the method of exercising includes engaging one or more handles to vary one or more of the distances. For example, in some embodiments, engaging the abdominal crunch exercise member includes grasping the handles 107 and pulling the device 100 toward the user, as in Figures 6A and 7B. This motion can correspond to an exercise traditionally performed as a cable row or on a rowing machine. The user's hands can be positioned such that his or her palms are facing up, down, or towards each other. When the user's palms face up, the user can exercise his or her lower abs, middle abs, side abs, lats, back and arms. When the user's palms face down or each other, the user can exercise the same or different muscles, and such exercises can have the same or different effects. For example, a user may primarily exercise triceps and shoulder muscles with palms facing down.

In some embodiments, the method of exercising includes overcoming a restoring force provided by a resistance member to vary a distance between one or more of the first and second thigh members 102a and 102b and chest contact member 101 to move one or more of the first and second thigh members 102a and 102b in a reciprocal motion. In some embodiments, the method includes the user engaging the first and second thigh members by lifting his or her legs to move one or more of the first and second thigh members in the reciprocal motion. In some embodiments, overcoming the restoring force provided by the resistance member is performed simultaneously with overcoming the restoring force provided by any one or more of the other resistance members described herein.

In some embodiments, the motions include pulling the apparatus towards the user, crunching the abdomen, adducting the thighs, and/or holding any or all of these motions. The user can perform these motions in any order, in any combination, or simultaneously. These motions can exercise most or all core muscles and the thighs. This allows a user to exercise these multiple muscle groups simultaneously using a single device. Simultaneously exercising these muscle groups allows the user to compound the effects of individual exercises compared to using no machine or traditional machines configured to exercise only a single muscle group at once.

In some embodiments, a user can use the apparatus in a seated position, lying on his or her back on the floor, in a hybrid seated/lying down position, or at an incline or decline. In accordance with various embodiments, use of the apparatus in a sitting position can provide a beginner exercise, use of the device in a lying down position can provide an intermediate exercise, and use of the device in a hybrid sitting/laying down position can provide an advanced exercise. Use of the apparatus in any of these positions can also alter the resistance and muscle groups used, thereby providing different workouts.

In addition, the putting pressure on the frontal abdominals using the apparatus can prevent lordosis or curvature of the lower back. This can reduce pressure on the lower back, critical to individuals with low back pain. As discussed above, a user can perform a "crunch" by crunching against the chest contact member 101. This can exercise the upper abs. It should also be noted that the figures illustrated the crunch motion in a rotational fashion; however, the crunch can also be performed at a straight angle. In other words, a user can push his or her chest so that the chest contact member 101 moves straight into the body of the device 100, as discussed above.

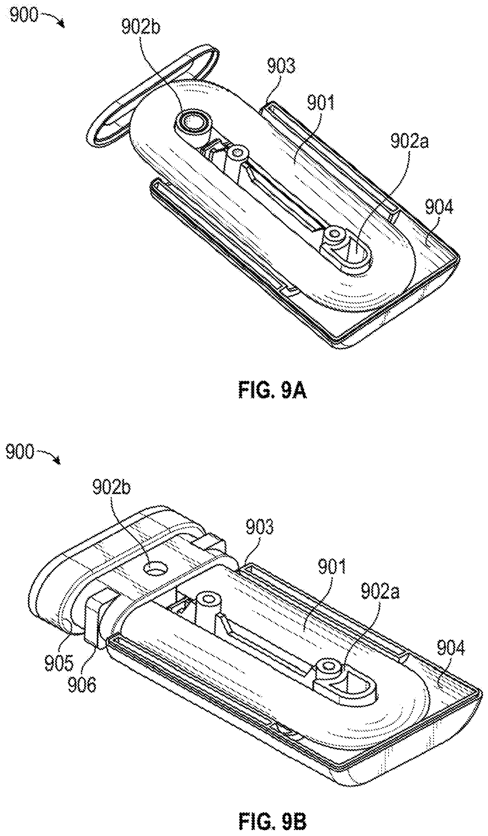

Figure 9A and Figure 9B represent isometric cutaway views of an exemplary resistance member 900 in accordance with an embodiment. In some embodiments, the resistance member comprises a first anchor 903, and a second anchor 905 configured to move in a reciprocal motion relative to the first anchor 903. In some embodiments, the resistance member comprises a spring 901. In some embodiments, the spring resists relative movement of the first and second anchor in a first direction of the reciprocal motion. In some embodiments, the spring aids relative movement of the first and second anchor in a direction of the reciprocal motion opposite to the first direction. In some embodiments, the reciprocal motion is between 5.08 cm and 45.72 cm [2 and 18 inches], between 10.16 cm and 40.64 cm [4 and 16 inches], between 12.7 cm and 30.48 cm [5 and 12 inches], or between 15.24 cm and 20.32 cm [6 and 8 inches]. In some embodiments, the reciprocal motion is 2.54 cm, 5.08 cm, 7.62 cm, 10.16 cm, 12.7 cm, 15.24 cm, 17.78 cm, 20.32 cm, or 22.86 cm [2, 3, 4, 5, 6, 7, 8, or 9 inches].

In some embodiments, the spring 901 can be connected to the first and second band connectors 902a and 902b such that the first and second band connectors are configured to move in a reciprocal motion. The reciprocal motion can include varying a distance between the first band connector 902a and second band connector 902b, or can include being adapted to vary a distance between the first band connector 902a and second band connector 902b. In some embodiments, varying a distance between the first band connector 902a and the second band connector 902b elongates the spring 901. In some embodiments, elongating the spring increases the tension on the spring, thereby increasing the resistance provided by the resistance member.

In some embodiments, the spring comprises a continuous band encircling a first band connector 902a and a second band connector 902b. In some embodiments, the continuous band is ring-shaped, circular, or ellipsoidal. In some embodiments, the band connector is a post or a hook. In some embodiments, the spring 901 comprises rubber or latex.

In some embodiments, the resistance member comprises a spacer between the first anchor and the second anchor that limits relative movement of the first and second anchor 903 and 905 in the direction opposite to the first direction and wherein the spring is in tension when the first and second anchor are at the minimum distance. In resistance member 900, the spacer comprises portions of the housing surrounding the first anchor 903 and the housing surrounding the second anchor 905. The housings abut one another to limit relative movement of the first and second anchor 903 and 905. Spring 901 is in tension when the housings abut one another.

In some embodiments, the spacer is configured to maintain a distance between the first anchor 903 and the second anchor 905 of between 0.3175 cm and 50.8 cm [1/8 of an inch and 20 inches], between 0.635 cm and 45.72 cm [1/4 of an inch and 18 inches], between 1.27 cm and 40.64 cm [1/2 of an inch and 16 inches], between 2.54 cm and 35.56 cm [1 inch and 14 inches], between 5.08 cm and 30.48 cm [2 inches and 12 inches], between 7.62 cm and 20.32 cm [3 and 8 inches], between 8.89 cm and 15.24 cm [3.5 and 6 inches], or between 10.16 cm and 12.7 cm [4 and 5 inches]. In some embodiments, the spacer is configured to maintain a distance between the first anchor and the second anchor of 0.3175 cm, 0.635 cm, 1.27 cm 2.54 cm, 5.08 cm, 7.62 cm, or 10.16 cm [1/8, 1/4, or 1/2 of an inch, or 1, 2, 3, or 4 inches].

In some embodiments, the first band connector 902a is connected to the first anchor 903. In some embodiments, the first anchor 903 comprises a lip, a rib, or an edge. In some embodiments, the first anchor 903 is part of a shell 904 or cover encasing at least a portion of the tension band. In some embodiments, the base connector can be configured to connect the resistance member to a chest contact member, chest crunch base, abdominal contact member, or abdominal crunch base such that the resistance member provides a restoring force when a user engages the device.

In some embodiments, the anchors 903 and 905 can be configured to connect the resistance member to a chest contact member, chest crunch base, abdominal contact member, or abdominal crunch base such that the resistance member provides a restoring force when a user engages the device. For example, in some embodiments the first anchor 903 is configured to connect the resistance member to a chest contact member and the second anchor 905 is configured to connect the resistance member to a chest crunch base. In some embodiments, the first anchor 903 is configured to connect the resistance member to a chest crunch base and the second anchor 905 is configured to connect the resistance member to a chest contact member. In some embodiments, the first anchor 903 is configured to connect the resistance member to an abdominal contact member and the second anchor 905 is configured to connect the resistance member to an abdominal crunch base. In some embodiments, the first anchor 903 is configured to connect the resistance member to an abdominal crunch base and the second anchor 905 is configured to connect the resistance member to an abdominal contact member.

In some embodiments, the resistance member includes a housing for containing the first anchor, second anchor, and the spring. In some embodiments, the housing has a length between 5.08 cm and 50.8 cm [2-20 inches], between 6.35 cm and 30.48 cm [2.5 and 12 inches], between 7.62 cm and 20.32 cm [3 and 8 inches], between 8.89 cm and 15.24 cm [3.5 and 6 inches], or between 10.16 cm and 12.7 cm [4 and 5 inches], a width of between 2.54 cm and 15.24 cm [1 and 6 inches], between 3.81 cm and 10.16 cm [1.5 and 4 inches], or between 5.08 cm and 7.62 cm [2 and 3 inches], and a height of between 1.27 cm and 7.62 cm [0.5 and 3 inches], or between 2.54 cm and 5.08 cm [1 and 2, inches[. In some embodiments the housing has a length of 10.16 cm [4 inches], a width of 5.08 cm [2 inches], and a height of 2.54 cm [1 inch].

In some embodiments, the housing comprises two parts: a first housing comprising first anchor 903 and second housing comprising second anchor 905, wherein at least one of the first and second anchors comprise an outer surface of the first and second housing. In some embodiments, the portion of the outer surface comprises a lip, a rib, or an edge. In some embodiments, at least one of the first and second anchor is part of a shell 904 or cover encasing at least a portion of the tension band.

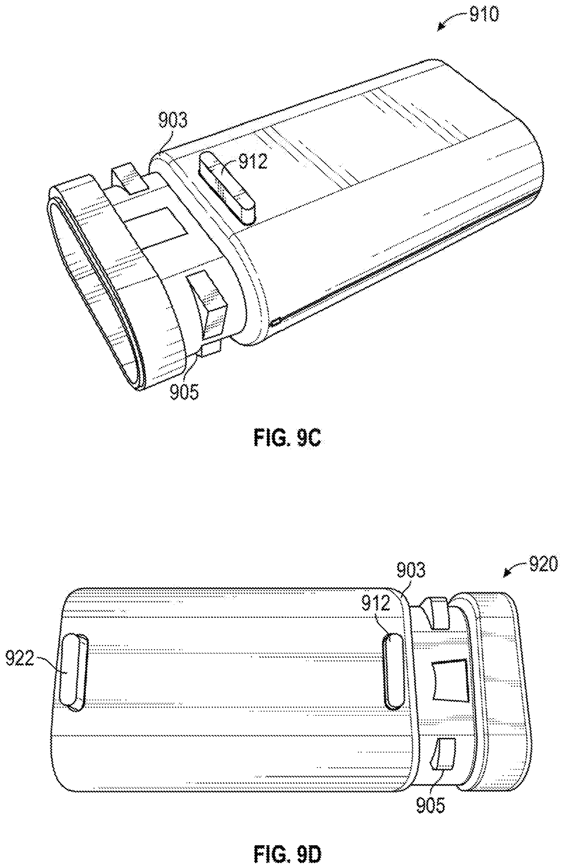

Figure 9C illustrates a resistance member 910 in accordance with another embodiment. Resistance member 910 comprises a third anchor 912 fixed relative to first anchor 903 and moveable relative to second anchor 905. In the embodiment of 910, third anchor 912 comprises a protrusion housing comprising the second and third anchor.

Figure 9D illustrates a resistance member 920 in accordance with another embodiment. Resistance member 920 comprises a fourth anchor 922 fixed relative to the third anchor 912. In the embodiment of 920, fourth anchor 922 comprises a protrusion from the housing that comprises the second, third, and fourth anchors.

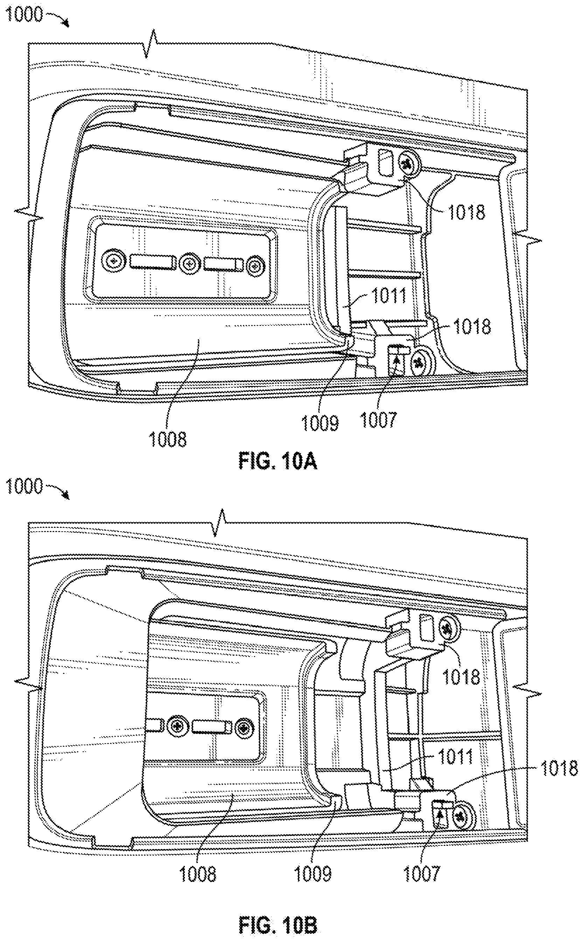

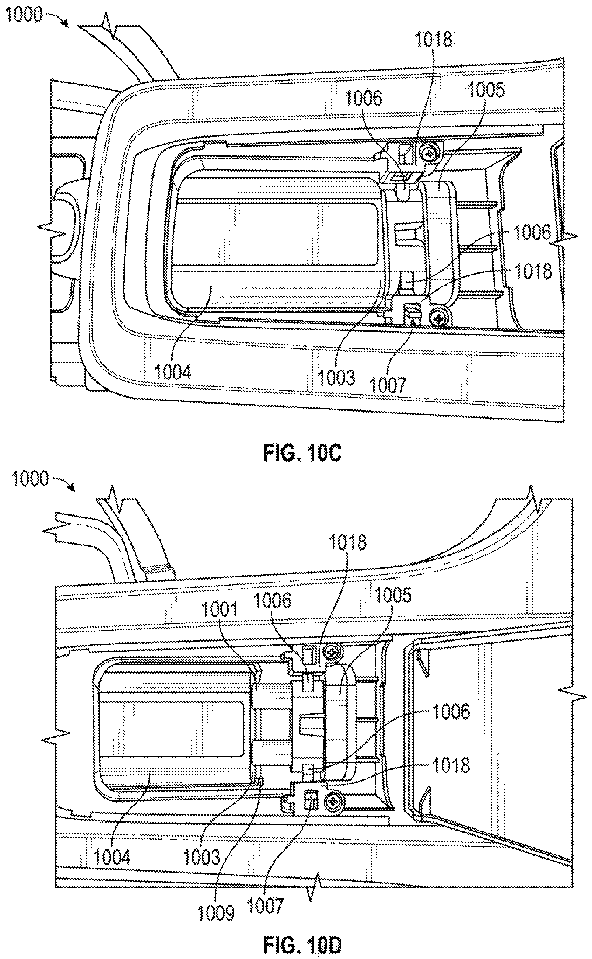

Figure 10A and Figure 10B represent top-down views of an exemplary resistance member compartment 1000 in accordance with an embodiment. Figure 10C and Figure 10D represent top-down views of an exemplary resistance member placed inside an exemplary resistance member compartment 1000. In some embodiments, the resistance member compartment comprises a bay 1008 configured to hold the shell 1004 of the resistance member. In some embodiments, the bay comprises a first edge, a lip, or a rib 1009 configured to engage the first anchor 1003. In some embodiments, the resistance member compartment 1000 comprises a second edge, a lip, or a rib 1011 configured to engage the second anchor 1005. In some embodiments, when the user compresses the module (e.g., the abdominal module, chest module, or thigh module), the resistance member connected to the first edge 1009 and the second edge 1011 by the first anchor 1003 and the second anchor 1005 provides a restoring force to the module.

In some embodiments, the resistance member compartment 1000 comprises a lock 1007 configured to lock the resistance member in place in the resistance member compartment. In some embodiments, the second anchor 1005 comprises a protrusion 1006 configured to engage a lock on the exercise apparatus 1007. In some embodiments, the lock 1007 engages with the protrusion 1006 to reversibly lock the resistance member in place during use. In some embodiments, the lock comprises a lever or a clip.

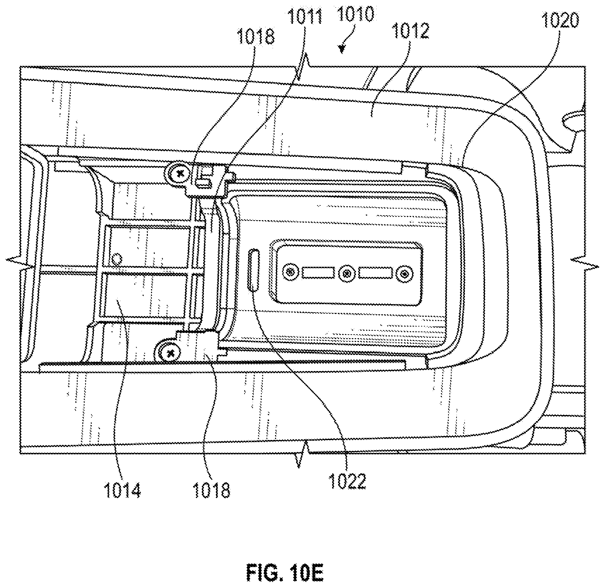

Figure 10E illustrates a resistance member compartment 1010 without a resistance member inserted, in accordance with an embodiment. Figure 10E illustrates a body contact member 1012 and a base 1014. The body contact member comprises a tube and the base comprises an insert. The insert moves in a reciprocal motion within the tube. The insert comprises a first anchor engagement 1009 and the tube comprises a second anchor engagement 1018, wherein the first anchor engagement moves relative to the second anchor engagement. In some embodiments, the second anchor engagement comprises an edge, a lip, or a rib 1011 configured to engage the second anchor 1005. In some embodiments, the second anchor engagement is a post 1018 configured to engage the second anchor 1005. In some embodiments, the second anchor engagement comprises a rib 1011 and one, two, or more posts 1018. The tube comprises an aperture 1020 sized to receive a resistance member (for example, the resistance members described above with respect to Figures 9A - 10D) that engages with the first anchor engagement and the second anchor engagement. Member compartment 1010 also includes an additional anchor engagement 1022 for a third anchor (for example, the third anchor embodiment described above with respect to Figure 9C).

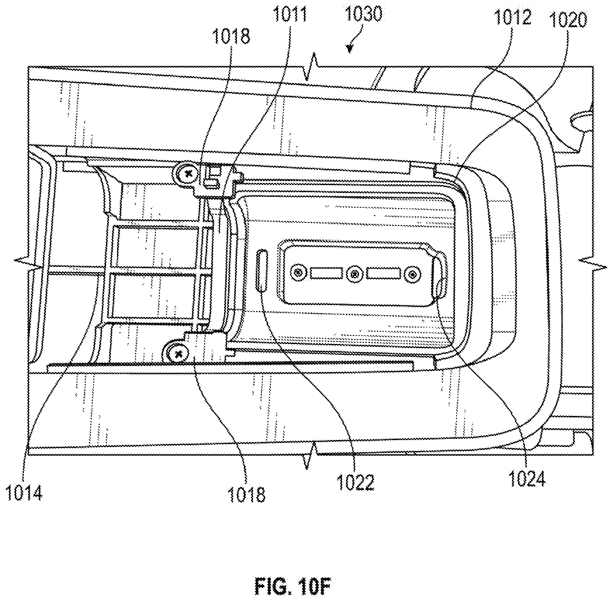

Figure 10F illustrates a resistance member compartment 1030 without a resistance member inserted, in accordance with an embodiment. Figure 10E illustrates a body contact member 1012 and a base 1014. The body contact member comprises a tube and the base comprises an insert. The insert moves in a reciprocal motion within the tube. The insert comprises a first anchor engagement 1009 and the tube comprises a second anchor engagement, which in some embodiments comprises a rib 1011 and/or one, two, or more posts 1018 configured to engage the second anchor, wherein the first anchor engagement moves relative to the second anchor engagement. The tube comprises an aperture 1020 sized to receive a resistance member (for example, the resistance members described above with respect to Figures 9A - 10D) that engages with the first anchor engagement and the second anchor engagement. Member compartment 1030 also includes an anchor engagement 1022 for a third anchor and an anchor engagement 1024 for a fourth anchor (for example, the fourth anchor embodiment described above with respect to Figure 9D).

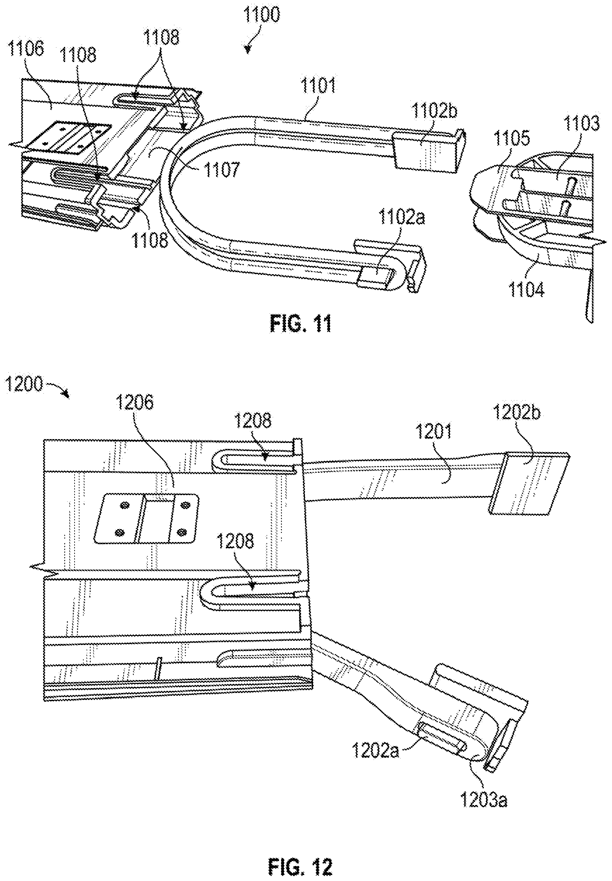

Figure 11 represents an isometric view of a disassembled exemplary resistance member 1100 in accordance with an embodiment. In some embodiments, the resistance member comprises a spring 1101. In some embodiments, the resistance member comprises a first spring 1102a connector and a second spring connector 1102b. In some embodiments, the distance between the first spring connector 1102a and second spring connector 1102b is fixed. Is some embodiments, a spring 1101 is connected to the first spring connector 1102a and second spring connector 1102b. In some embodiments, the resistance member comprises a paddle 1103 configured to engage the spring. In some embodiments, the paddle 1103 is configured to move in a reciprocal motion relative to the first and second spring connectors 1102a and 1102b. In some embodiments, the spring 1101 resists relative movement between the paddle and the first and second anchors in a first direction of the reciprocal motion. In some embodiments, the spring 1101 aids relative movement between the paddle 1103 and the first and second spring connectors 1102a and 1102b in a direction of the reciprocal motion opposite the first direction. In some embodiments, the reciprocal motion is between 5.08 cm and 45.72 cm [2 and 18 inches], between 10.16 cm and 40.64 cm [4 and 16 inches], between 12.7 cm and 30.48 cm [5 and 12 inches], or between 15.24 cm and 20.32 cm [6 and 8 inches]. In some embodiments, the reciprocal motion is 2.54 cm, 5.08 cm, 7.62 cm, 10.16 cm, 12.7 cm, 15.24 cm, 17.78 cm, 20.32 cm, or 22.86 cm [2, 3, 4, 5, 6, 7, 8, or 9 inches].

In some embodiments, the spring 1101 comprises rubber or latex. In some embodiments, the spring 1101 comprises a continuous band. In some embodiments, the continuous band is ring-shaped, circular, or ellipsoidal. In some embodiments, the continuous band is folded over each of the first and second spring connectors 1102a and 1102b, thereby forming a U or semi-circular shape. In some embodiments, the paddle comprises a rim or a curved edge 1104. In some embodiments, the paddle comprises one or more guides on each side 1105. In some embodiments, the rim or curved edge 1104 contacts the spring 1101. In some embodiments, varying a distance between an end of the paddle and one of the one or more spring connectors 1102a and 1102b elongates the spring 1101. In some embodiments, elongating the spring 1101 increases the tension on the spring 1101, thereby increasing the resistance of the resistance member.

In some embodiments, resistance member 1100 further comprises a cover 1106. In some embodiments, the cover comprises a tube, wherein the spring is configured to move in a reciprocal motion within the tube. In some embodiments, the tube comprises a hollow internal space comprising an inner wall 1107. In some embodiments, the cover comprises slots 1108 configured to engage the spring connectors 1102a and 1102b.

In some embodiments, one of an abdominal contact member or an abdominal crunch base comprises or is connected to the paddle and the other of the abdominal crunch member or abdominal crunch bases comprises or is connected to the first and second spring connector. In some embodiments, the other of the abdominal crunch member or abdominal crunch bases comprises or is permanently or removably connected to the cover 1106, which comprises or is connected to the first and second spring connectors. In some embodiments, one of a chest contact member or a chest crunch base comprises or is connected to the paddle and the other of the chest crunch member or chest crunch bases comprises or is connected to the first and second spring connector. In some embodiments, a first thigh member comprises or is connected to the paddle and a second thigh member comprises or is connected to the first and second spring connector. Thus, in some embodiments, the spring can provide a restoring force on a module when a user engages the device.

Figure 12 represents an isometric view of a disassembled exemplary resistance member 1200 in accordance with an embodiment. In some embodiments, the resistance member comprises a spring 1201. In some embodiments, the spring comprises a linear band. In some embodiments, the spring comprises latex or rubber. In some embodiments, the resistance member comprises a first spring 1202a connector and a second spring connector 1202b. In some embodiments, the first and second spring connectors 1202a and 1202b comprise posts or hooks. In some embodiments, the linear band comprises loops at one or more ends of the band, such as 1203a, that engage with a spring connector. In some embodiments, the loops can be reinforced with additional latex or rubber.

In some embodiments, the springs 1001, 1101, and 1201 are interchangeable. In some embodiments, varying the length, elasticity, or thickness of a spring can vary the resistance the tension band can provide. In some embodiments, the springs provide between 2.27 kg and 72.57 kg [5 and 160 pounds] of resistance. In some embodiments, the spring provides 2.27 kg, 4.54 kg, 6.80 kg, 9.07 kg, 11.34 kg, 13.61 kg, 15.88 kg, 18.14 kg, 20.41 kg, 22.68 kg, 24.95 kg, 27.22 kg, 29.48 kg, 31.75 kg, 36.29 kg, 40.82 kg, 45.36 kg, 49.90 kg, 54.43 kg, 58.97 kg, 63.50 kg, 68.04 kg, or 72.57 kg [5, 10, 15, 20, 25, 30, 35, 40, 45, 50, 55, 60, 65, 70, 80, 90, 100, 110, 120, 130, 140, 150, or 160 pounds] of resistance.

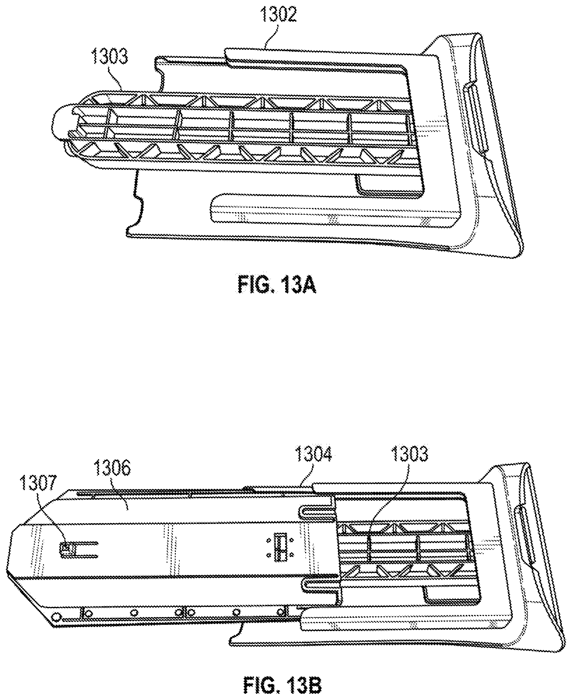

Figures 13A and 13B illustrate abdominal contact members configured to be used with the springs of Figures 11 and 12. The abdominal contact member in Figure 13A includes a wider base 1302 than the base 1304 in Figure 13B. Each of the abdominal contact members comprises a paddle 1303. Figure 13B also illustrates a cover 1306 configured to removably engage with an abdominal contact base. The cover comprises a lever with a nub 1307, which can secure the cover 1306 to the abdominal contact base.

Figure 14 illustrates an exercise apparatus comprising a chest contact member 1402 adapted to receive a chest crunch base, including the exemplary chest crunch base 103 depicted in Figure 1A. The chest crunch base comprises an insert and the chest contact member comprises a tube, wherein the insert is configured to move in a reciprocal motion within the tube. The tube comprises a hollow internal space comprising an inner wall 1406. The insert comprises an outer wall. In some embodiments, the outer wall of the chest crunch base comprises a rail. The tube includes complimentary guides 1410a and 1410b to prevent contact between the inner wall of the tube and outer wall of the chest crunch base during the reciprocal motion. In some embodiments, the complementary guides 1410a and 1410b engage and guide the rail of the chest crunch base. In some embodiments, the complementary guides 1410a and 1410b reduce friction or vibration caused by the reciprocal motion between the chest contact member and the chest contact base.

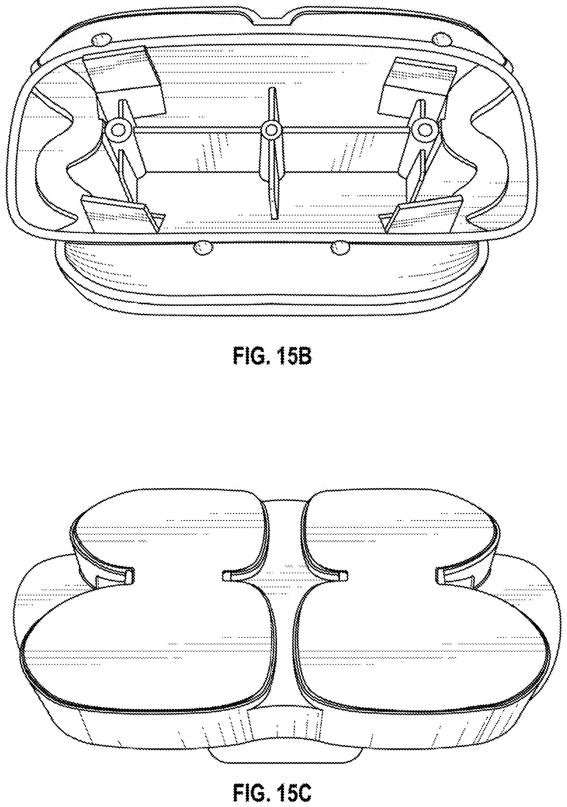

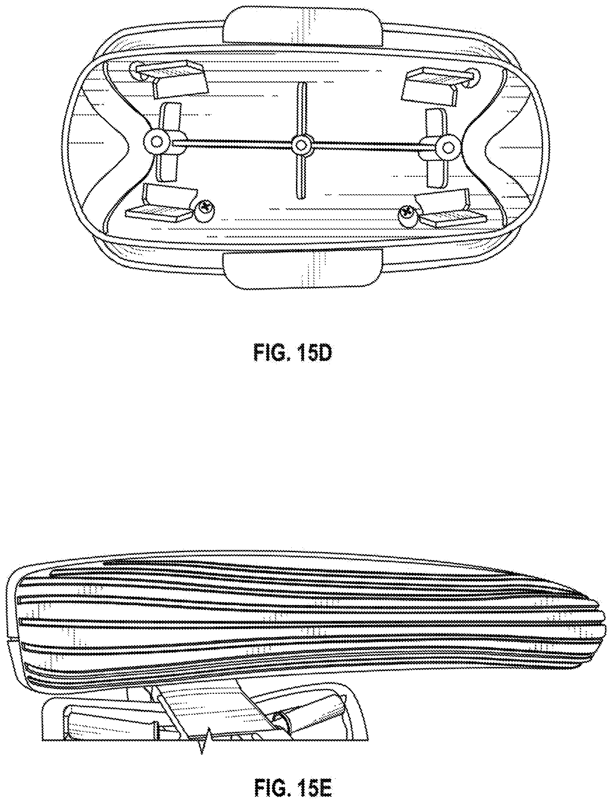

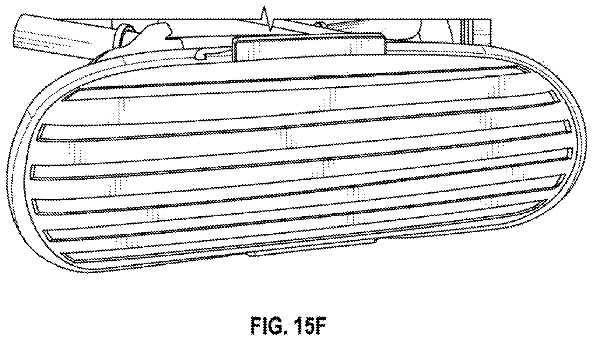

Figures 15A to 15F illustrate exemplary contact members and backings for abdominal contact members and chest contact members in accordance with some embodiments. Figure 15A illustrates an abdominal contact member with six contact protrusions in accordance with an embodiment. Figure 15B illustrates a backing for the abdominal contact member of Figure 15A. Figure 15C illustrates an abdominal contact member with four contact protrusions in accordance with an embodiment. Figure 15D illustrates a backing for the abdominal contact member of Figure 15C. Figure 15E illustrates a chest contact member in accordance with an embodiment. In some embodiments, the chest contact member is textured. In some embodiments, the chest contact member comprises grooves. In some embodiments, the chest contact member is curved or concave. The curve or concave shape can be adapted to contact a user's chest. Figure 15F illustrates a chest contact member in accordance with an embodiment. In some embodiments, the chest contact member is not concave, and is flat or substantially flat relative to the length, the width, or both the length and width of the chest contact member.

Through contact prevention, the complimentary guides may allow for freer relative motion of the base and body contact member during the reciprocal motion. Further, by preventing contact, the complimentary guides may allow for a longer life of the base and body contact member. In some embodiments, the complimentary guides may reduce an angular range of motion of the tube relative to the insert. In some embodiments, the complementary guides comprise a bearing and a female guide rail.

Although the disclosed embodiments have been fully described with reference to the accompanying drawings, it is to be noted that various changes and modifications will become apparent to those skilled in the art. The scope of the invention is defined by the appended claims.

## Claims

1. An exercise apparatus (100, 200, 400, 500) comprising:
a first thigh member (102a, 202a, 402a, 502a) and a second thigh member (102b, 202b, 402b, 502b) moving in a first reciprocal motion when the user engages the first and second thigh members (102a, 102b, 202a, 202b, 402a, 402b, 502a, 502b), the first reciprocal motion varying a first distance between the first and second thigh members (102a, 102b, 202a, 202b, 402a, 402b, 502a, 502b); and
a chest contact member (101, 201, 401, 501) moving in a second reciprocal motion when a user engages the chest contact member (101, 201, 401, 501), the second reciprocal motion varying a second distance between the chest contact member (101, 201, 401, 501) and the first and second thigh members (102a, 102b, 202a, 202b, 402a, 402b, 502a, 502b),
**characterized in that** the exercise apparatus (100, 200, 400, 500) further comprises an abdominal contact member (110, 210, 410, 510) moving in a third reciprocal motion when the user engages the abdominal contact member (110, 210, 410, 510), the third reciprocal motion varying a third distance between the abdominal contact member (110, 210, 410, 510) and the first and second thigh members (102a, 102b, 202a, 202b, 402a, 402b, 502a, 502b).

2. The exercise apparatus (100, 200, 400, 500) of claim 1, further comprising a first resistance member, wherein the first resistance member provides a restoring force on the first thigh member (102a, 202a, 402a, 502a) relative to the second thigh member (102b, 202b, 402b, 502b) when the first distance decreases.

3. The exercise apparatus (100, 200, 400, 500) of claim 1 or 2, wherein a maximum distance between the first and second thigh members (102a, 102b, 202a, 202b, 402a, 402b, 502a) is adjustable.

4. The exercise apparatus (100, 200, 400, 500) of any of claims 1-3, wherein the first reciprocal motion comprises both the first thigh member (102a, 202a, 402a, 502a) and the second thigh member (102b, 202b, 402b, 502b) moving relative to a centerline of the exercise apparatus (100, 200, 400, 500).

5. The exercise apparatus (100, 200, 400, 500) of any of claims 1-4, further comprising a second resistance member, wherein the second resistance member provides a restoring force on the chest contact member (101, 201, 401, 501) when the second distance decreases.

6. The exercise apparatus (100, 200, 400, 500) of any of claims 1-5, further comprising an abdominal crunch base guiding the abdominal contact member (110, 210, 410, 510) when the abdominal contact member (110, 210, 410, 510) moves in the third reciprocal motion.

7. The exercise apparatus (100, 200, 400, 500) of claim 6, further comprising a third resistance member, wherein the third resistance member provides a restoring force on the abdominal contact member (110, 210, 410, 510) when the third distance decreases.

8. The exercise apparatus (100, 200, 400, 500) of any of claims 1-7, wherein the first and second thigh members (102a, 102b, 202a, 202b, 402a, 402b, 502a, 502b) are further configured to move in a fourth reciprocal motion when the user engages the first and second thigh members (102a, 102b, 202a, 202b, 402a, 402b, 502a, 502b), the fourth reciprocal motion varying a fourth distance between the chest contact member (101, 201, 401, 501) and one or more of the first and second thigh members (102a, 102b, 202a, 202b, 402a, 402b, 502a, 502b).

9. The exercise apparatus (100, 200, 400, 500) of any of claim 8, further comprising a fourth resistance member, wherein the fourth resistance member provides a restoring force on the one or more of the first and second thigh members (102a, 102b, 202a, 202b, 402a, 402b, 502a, 502b) relative to the chest contact member (101, 201, 401, 501) when the fourth distance decreases.

10. The exercise apparatus (100, 200, 400, 500) of any of claims 2-9, wherein the restoring force of one or more of the resistance members is adjustable and/or wherein one or more of the resistance members are interchangeable with other resistance members.

11. A method of exercising using an exercise apparatus (100, 200, 400, 500) comprising:
engaging a first thigh member (102a, 202a, 402a, 502a) and a second thigh member (102b, 202b, 402b, 502b);
engaging a chest contact member (101, 201, 401, 501), wherein a chest crunch comprises the chest contact member (101, 201, 401, 501);
overcoming a restoring force provided by a first resistance member to vary a first distance between the first and second thigh members (102a, 102b, 202a, 202b, 402a, 402b, 502a, 502b) to move the first and second thigh members (102a, 102b, 202a, 202b, 402a, 402b, 502a, 502b) in a first reciprocal motion; and
overcoming a restoring force provided by a second resistance member to vary a second distance between the chest contact member (101, 201, 401, 501) and one or more of the first and second thigh members (102a, 102b, 202a, 202b, 402a, 402b, 502a, 502b) to move the chest contact member (101, 201, 4011 501) in a second reciprocal motion, wherein overcoming the restoring force provided by the first resistance member is performed simultaneously with overcoming the restoring force provided by the second resistance member,
**characterized in that** the method further comprises:
engaging an abdominal contact member (110, 210, 410, 510); and
overcoming a restoring force provided by a third resistance member to vary a third distance between the abdominal contact member (110, 210, 410, 510) and one or more of the first and second thigh members (102a, 102b, 202a, 202b, 402a, 402b, 502a, 502b) to move the abdominal contact member (110, 210, 410, 510) in a third reciprocal motion, wherein overcoming the restoring force provided by the third resistance member is performed simultaneously with overcoming the restoring force provided by the first and second resistance members.

12. The method of claim 11, wherein overcoming the restoring force provided by the second resistance member or the third resistance member further comprises engaging one or more handles to pull the exercise apparatus (100, 200, 400, 500) towards a user's body.

13. The method of claim 11 or 12, further comprising overcoming a restoring force provided by a fourth resistance member to vary a fourth distance between one or more of the first and second thigh members (102a, 102b, 202a, 202b, 402a, 402b, 502a, 502b) and chest contact member (101, 201, 401, 501) to move one or more of the first and second thigh members (102a, 102b, 202a, 202b, 402a, 402b, 502a, 502b) in a fourth reciprocal motion, wherein overcoming the restoring force provided by the fourth resistance member is performed simultaneously with overcoming the restoring force provided by the first and second resistance members.

## Patentansprüche

1. Übungsgerät (100, 200, 400, 500), aufweisend:
ein erstes Oberschenkelelement (102a, 202a, 402a, 502a) und ein zweites Oberschenkelelement (102b, 202b, 402b, 502b), die sich in einer ersten Hin- und Herbewegung bewegen, wenn der Benutzer mit dem ersten und dem zweiten Oberschenkelelement (102a, 102b, 202a, 202b, 402a, 402b, 502a, 502b) zusammenwirkt, wobei die erste Hin und Herbewegung einen ersten Abstand zwischen dem ersten und dem zweiten Oberschenkelelement (102a, 102b, 202a, 202b, 402a, 402b, 502a, 502b) verändert; und
ein Brustkontaktelement (101, 201, 401, 501), das sich in einer zweiten Hin- und Herbewegung bewegt, wenn ein Benutzer mit dem Brustkontaktelement (101, 201, 401, 501) zusammenwirkt, wobei die zweite Hin- und Herbewegung einen zweiten Abstand zwischen dem Brustkontaktelement (101, 201, 401, 501) und dem ersten und zweiten Oberschenkelelement (102a, 102b, 202a, 202b, 402a, 402b, 502a, 502b) verändert,
**dadurch gekennzeichnet, dass** das Übungsgerät (100, 200, 400, 500) ferner ein Bauchkontaktelement (110, 210, 410, 510) aufweist, das sich in einer dritten Hin- und Herbewegung bewegt, wenn der Benutzer mit dem Bauchkontaktelement (110, 210, 410, 510) zusammenwirkt, wobei die dritte Hin- und Herbewegung einen dritten Abstand zwischen dem Bauchkontaktelement (110, 210, 410, 510) und dem ersten und zweiten Oberschenkelelement (102a, 102b, 202a, 202b, 402a, 402b, 502a, 502b) verändert.

2. Übungsgerät (100, 200, 400, 500) nach Anspruch 1, ferner aufweisend ein erstes Widerstandselement, wobei das erste Widerstandselement eine Rückstellkraft auf das erste Oberschenkelelement (102a, 202a, 402a, 502a) relativ zu dem zweiten Oberschenkelelement (102b, 202b, 402b, 502b) bereitstellt, wenn der erste Abstand abnimmt.

3. Übungsgerät (100, 200, 400, 500) nach Anspruch 1 oder 2, wobei ein maximaler Abstand zwischen dem ersten und zweiten Oberschenkelelement (102a, 102b, 202a, 202b, 402a, 402b, 502a) einstellbar ist.

4. Übungsgerät (100, 200, 400, 500) nach einem der Ansprüche 1 bis 3, wobei die erste Hin- und Herbewegung aufweist, dass sowohl das erste Oberschenkelelement (102a, 202a, 402a, 502a) als auch das zweite Oberschenkelelement (102b, 202b, 402b, 502b) sich relativ zu einer Mittellinie des Übungsgeräts (100, 200, 400, 500) bewegen.

5. Übungsgerät (100, 200, 400, 500) nach einem der Ansprüche 1-4, das ferner ein zweites Widerstandselement aufweist, wobei das zweite Widerstandselement eine Rückstellkraft auf das Brustkontaktelement (101, 201, 401, 501) bereitstellt, wenn sich der zweite Abstand verringert.

6. Übungsgerät (100, 200, 400, 500) nach einem der Ansprüche 1 bis 5, das ferner eine abdominale Crunch-Basis aufweist, die das Bauchkontaktelement (110, 210, 410, 510) führt, wenn sich das Bauchkontaktelement (110, 210, 410, 510) in der dritten Hin- und Herbewegung bewegt.

7. Übungsgerät (100, 200, 400, 500) nach Anspruch 6, das ferner ein drittes Widerstandselement aufweist, wobei das dritte Widerstandselement eine Rückstellkraft auf das Bauchkontaktelement (110, 210, 410, 510) bereitstellt, wenn der dritte Abstand abnimmt.

8. Übungsgerät (100, 200, 400, 500) nach einem der Ansprüche 1 bis 7, wobei das erste und das zweite Oberschenkelelement (102a, 102b, 202a, 202b, 402a, 402b, 502a, 502b) ferner so konfiguriert sind, dass sie sich in einer vierten Hin- und Herbewegung bewegen, wenn der Benutzer mit dem ersten und dem zweiten Oberschenkelelement (102a, 102b, 202a, 202b, 402a, 402b, 502a, 502b) zusammenwirkt, wobei die vierte Hin- und Herbewegung einen vierten Abstand zwischen dem Brustkontaktelement (101, 201, 401, 501) und dem ersten und/oder dem zweiten Oberschenkelelement (102a, 102b, 202a, 202b, 402a, 402b, 502a, 502b) verändert.

9. Übungsgerät (100, 200, 400, 500) nach Anspruch 8, ferner aufweisend ein viertes Widerstandselement, wobei das vierte Widerstandselement eine Rückstellkraft auf das erste und/oder das zweite Oberschenkelelement (102a, 102b, 202a, 202b, 402a, 402b, 502a, 502b) relativ zu dem Brustkontaktelement (101, 201, 401, 501) bereitstellt, wenn der vierte Abstand abnimmt.

10. Übungsgerät (100, 200, 400, 500) nach einem der Ansprüche 2-9, wobei die Rückstellkraft eines oder mehrerer der Widerstandselemente einstellbar ist und/oder wobei ein oder mehrere der Widerstandselemente mit anderen Widerstandselementen austauschbar sind.

11. Verfahren zum Trainieren unter Verwendung eines Übungsgeräts (100, 200, 400, 500), aufweisend:
Zusammenwirken mit einem ersten Oberschenkelelement (102a, 202a, 402a, 502a) und einem zweiten Oberschenkelelement (102b, 202b, 402b, 502b);
Zusammenwirken mit einem Brustkontaktelement (101, 201, 401, 501), wobei ein Brustcrunch das Brustkontaktelement (101, 201, 401, 501) aufweist;
Überwinden einer Rückstellkraft, die durch ein erstes Widerstandselement bereitgestellt wird, um einen ersten Abstand zwischen dem ersten und dem zweiten Oberschenkelelement (102a, 102b, 202a, 202b, 402a, 402b, 502a, 502b) zu verändern, um das erste und das zweite Oberschenkelelement (102a, 102b, 202a, 202b, 402a, 402b, 502a, 502b) in einer ersten Hin- und Herbewegung zu bewegen; und
Überwinden einer Rückstellkraft, die von einem zweiten Widerstandselement bereitgestellt wird, um einen zweiten Abstand zwischen dem Brustkontaktelement (101, 201, 401, 501) und dem ersten und/oder dem zweiten Oberschenkelelement (102a, 102b, 202a, 202b, 402a, 402b, 502a, 502b) zu variieren, um das Brustkontaktelement (101, 201, 401, 501) in einer zweiten Hin- und Herbewegung zu bewegen, wobei das Überwinden der von dem ersten Widerstandselement bereitgestellten Rückstellkraft gleichzeitig mit dem Überwinden der von dem zweiten Widerstandselement bereitgestellten Rückstellkraft durchgeführt wird,
**dadurch gekennzeichnet, dass** das Verfahren weiterhin aufweist:
Zusammenwirken mit einem Bauchkontaktelement (110, 210, 410, 510); und
Überwinden einer Rückstellkraft, die durch ein drittes Widerstandselement bereitgestellt wird, um einen dritten Abstand zwischen dem Bauchkontaktelement (110, 210, 410, 510) und dem ersten und/oder dem zweiten Oberschenkelelement (102a, 102b, 202a, 202b, 402a, 402b, 502a, 502b) zu variieren, um das Bauchkontaktelement (110, 210, 410, 510) in einer dritten Hin- und Herbewegung zu bewegen, wobei das Überwinden der durch das dritte Widerstandselement bereitgestellten Rückstellkraft gleichzeitig mit dem Überwinden der durch das erste und zweite Widerstandselement bereitgestellten Rückstellkraft durchgeführt wird.

12. Verfahren nach Anspruch 11, wobei das Überwinden der Rückstellkraft, die durch das zweite Widerstandselement oder das dritte Widerstandselement bereitgestellt wird, ferner das Ergreifen eines oder mehrerer Griffe aufweist, um das Übungsgerät (100, 200, 400, 500) zum Körper eines Benutzers zu ziehen.

13. Verfahren nach Anspruch 11 oder 12, ferner aufweisend das Überwinden einer Rückstellkraft, die von einem vierten Widerstandselement bereitgestellt wird, um einen vierten Abstand zwischen dem ersten und/oder dem zweiten Oberschenkelelement (102a, 102b, 202a, 202b, 402a, 402b, 502a, 502b) und dem Brustkontaktelement (101, 201, 401, 501) zu variieren, um das erste und/oder das zweite Oberschenkelelement (102a, 102b, 202a, 202b, 402a, 402b, 502a, 502b) in einer vierten Hin- und Herbewegung zu bewegen, wobei das Überwinden der Rückstellkraft, die durch das vierte Widerstandselement bereitgestellt wird, gleichzeitig mit dem Überwinden der Rückstellkraft, die durch die ersten und zweiten Widerstandselemente bereitgestellt wird, durchgeführt wird.

## Revendications

1. Appareil d'exercice (100, 200, 400, 500) comprenant :
un premier élément de cuisse (102a, 202a, 402a, 502a) et un second élément de cuisse (102b, 202b, 402b, 502b) se déplaçant dans un premier mouvement de va-et-vient lorsque l'utilisateur vient en prise avec les premier et second éléments de cuisse (102a, 102b, 202a, 202b, 402a, 402b, 502a, 502b), le premier mouvement de va-et-vient faisant varier une première distance entre les premier et second éléments de cuisse (102a, 102b, 202a, 202b, 402a, 402b, 502a, 502b) ; et
un élément de contact thoracique (101, 201, 401, 501) se déplaçant dans un deuxième mouvement de va-et-vient lorsqu'un utilisateur vient en prise avec l'élément de contact thoracique (101, 201, 401, 501), le deuxième mouvement de va-et-vient faisant varier une deuxième distance entre l'élément de contact thoracique (101, 201, 401, 501) et les premier et second éléments de cuisse (102a, 102b, 202a, 202b, 402a, 402b, 502a, 502b),
**caractérisé en ce que** l'appareil d'exercice (100, 200, 400, 500) comprend en outre un élément de contact abdominal (110, 210, 410, 510) se déplaçant dans un troisième mouvement de va-et-vient lorsque l'utilisateur vient en prise avec l'élément de contact abdominal (110, 210, 410, 510), le troisième mouvement de va-et-vient faisant varier une troisième distance entre l'élément de contact abdominal (110, 210, 410, 510) et les premier et second éléments de cuisse (102a, 102b, 202a, 202b, 402a, 402b, 502a, 502b).

2. Appareil d'exercice (100, 200, 400, 500) selon la revendication 1, comprenant en outre un premier élément de résistance, le premier élément de résistance fournissant une force de rappel sur le premier élément de cuisse (102a, 202a, 402a, 502a) par rapport au second élément de cuisse (102b, 202b, 402b, 502b) lorsque la première distance diminue.

3. Appareil d'exercice (100, 200, 400, 500) selon la revendication 1 ou 2, une distance maximale entre les premier et second éléments de cuisse (102a, 102b, 202a, 202b, 402a, 402b, 502a) étant réglable.

4. Appareil d'exercice (100, 200, 400, 500) selon l'une quelconque des revendications 1 à 3, le premier mouvement de va-et-vient comprenant à la fois le premier élément de cuisse (102a, 202a, 402a, 502a) et le second élément de cuisse (102b, 202b, 402b, 502b) se déplaçant par rapport à une ligne centrale de l'appareil d'exercice (100, 200, 400, 500).

5. Appareil d'exercice (100, 200, 400, 500) selon l'une quelconque des revendications 1 à 4, comprenant en outre un deuxième élément de résistance, le deuxième élément de résistance fournissant une force de rappel sur l'élément de contact thoracique (101, 201, 401, 501) lorsque la deuxième distance diminue.

6. Appareil d'exercice (100, 200, 400, 500) selon l'une quelconque des revendications 1 à 5, comprenant en outre une base de redressement partiel abdominal guidant l'élément de contact abdominal (110, 210, 410, 510) lorsque l'élément de contact abdominal (110, 210, 410, 510) se déplace dans le troisième mouvement de va-et-vient.

7. Appareil d'exercice (100, 200, 400, 500) selon la revendication 6, comprenant en outre un troisième élément de résistance, le troisième élément de résistance fournissant une force de rappel sur l'élément de contact abdominal (110, 210, 410, 510) lorsque la troisième distance diminue.

8. Appareil d'exercice (100, 200, 400, 500) selon l'une quelconque des revendications 1 à 7, les premier et second éléments de cuisse (102a, 102b, 202a, 202b, 402a, 402b, 502a, 502b) étant en outre configurés pour se déplacer dans un quatrième mouvement de va-et-vient lorsque l'utilisateur vient en prise avec les premier et second éléments de cuisse (102a, 102b, 202a, 202b, 402a, 402b, 502a, 502b), le quatrième mouvement de va-et-vient faisant varier une quatrième distance entre l'élément de contact thoracique (101, 201, 401, 501) et un ou plusieurs des premier et second éléments de cuisse (102a, 102b, 202a, 202b, 402a, 402b, 502a, 502b).

9. Appareil d'exercice (100, 200, 400, 500) selon l'une quelconque des revendications 8, comprenant en outre un quatrième élément de résistance, le quatrième élément de résistance fournissant une force de rappel sur le ou les premier et second éléments de cuisse (102a, 102b, 202a, 202b, 402a, 402b, 502a, 502b) par rapport à l'élément de contact thoracique (101, 201, 401, 501) lorsque la quatrième distance diminue.

10. Appareil d'exercice (100, 200, 400, 500) selon l'une quelconque des revendications 2 à 9, la force de rappel d'un ou plusieurs des éléments de résistance étant réglable et/ou un ou plusieurs des éléments de résistance étant interchangeables avec d'autres éléments de résistance.

11. Procédé d'exercice utilisant un appareil d'exercice (100, 200, 400, 500) comprenant :
la mise en prise d'un premier élément de cuisse (102a, 202a, 402a, 502a) et d'un second élément de cuisse (102b, 202b, 402b, 502b) ;
la mise en prise d'un élément de contact thoracique (101, 201, 401, 501), un relevé partiel de buste comprenant l'élément de contact thoracique (101, 201, 401, 501) ;
le fait de surmonter une force de rappel fournie par un premier élément de résistance pour faire varier une première distance entre les premier et second éléments de cuisse (102a, 102b, 202a, 202b, 402a, 402b, 502a, 502b) pour déplacer les premier et second éléments de cuisse (102a, 102b, 202a, 202b, 402a, 402b, 502a, 502b) dans un premier mouvement de va-et-vient ; et
le fait de surmonter une force de rappel fournie par un deuxième élément de résistance pour faire varier une deuxième distance entre l'élément de contact thoracique (101, 201, 401, 501) et un ou plusieurs des premier et second éléments de cuisse (102a, 102b, 202a, 202b, 402a, 402b, 502a, 502b) pour déplacer l'élément de contact thoracique (101, 201, 4011 501) dans un deuxième mouvement de va-et-vient, le fait de surmonter la force de rappel fournie par le premier élément de résistance étant réalisé simultanément au fait de surmonter la force de rappel fournie par le deuxième élément de résistance,
**caractérisé en ce que** le procédé comprend en outre :
la mise en prise d'un élément de contact abdominal (110, 210, 410, 510) ; et
le fait de surmonter une force de rappel fournie par un troisième élément de résistance pour faire varier une troisième distance entre l'élément de contact abdominal (110, 210, 410, 510) et un ou plusieurs des premier et second éléments de cuisse (102a, 102b, 202a, 202b, 402a, 402b, 502a, 502b) pour déplacer l'élément de contact abdominal (110, 210, 410, 510) dans un troisième mouvement de va-et-vient, le fait de surmonter la force de rappel fournie par le troisième élément de résistance étant réalisé simultanément au fait de surmonter la force de rappel fournie par les premier et deuxième éléments de résistance.

12. Procédé selon la revendication 11, le fait de surmonter la force de rappel fournie par le deuxième élément de résistance ou le troisième élément de résistance comprenant en outre la mise en prise d'une ou plusieurs poignées pour tirer l'appareil d'exercice (100, 200, 400, 500) vers le corps d'un utilisateur.

13. Procédé selon la revendication 11 ou 12, comprenant en outre le fait de surmonter une force de rappel fournie par un quatrième élément de résistance pour faire varier une quatrième distance entre un ou plusieurs des premier et second éléments de cuisse (102a, 102b, 202a, 202b, 402a, 402b, 502a, 502b) et l'élément de contact thoracique (101, 201, 401, 501) pour déplacer un ou plusieurs des premier et second éléments de cuisse (102a, 102b, 202a, 202b, 402a, 402b, 502a, 502b) dans un quatrième mouvement de va-et-vient, le fait de surmonter la force de rappel fournie par le quatrième élément de résistance étant réalisé simultanément au fait de surmonter la force de rappel fournie par les premier et deuxième éléments de résistance.
